(19) Europäisches Patentamt / European Patent Office / Office européen des brevets

(11) **EP 4 339 811 B1**

(12) **EUROPEAN PATENT SPECIFICATION**

(45) Date of publication and mention
of the grant of the patent:
**08.07.2026 Bulletin 2026/28**

(21) Application number: **23197177.1**

(22) Date of filing: **13.09.2023**

(51) International Patent Classification (IPC):
**G06F 18/25** *(2023.01)* **G06F 18/2431** *(2023.01)*
**G06F 18/21** *(2023.01)*

(52) Cooperative Patent Classification (CPC):
**G06F 18/254; G06F 18/2193; G06F 18/2431**

(54) **METHOD AND SYSTEM FOR EVALUATING CLINICAL EFFICACY OF MULTI-LABEL MULTI-CLASS COMPUTATIONAL DIAGNOSTIC MODELS**

VERFAHREN UND SYSTEM ZUR BEWERTUNG DER KLINISCHEN WIRKSAMKEIT VON MEHRMARKIERTEN MEHRKLASSIGEN COMPUTERGESTÜTZTEN DIAGNOSEMODELLEN

PROCÉDÉ ET SYSTÈME D'ÉVALUATION DE L'EFFICACITÉ CLINIQUE DE MODÈLES DE DIAGNOSTIC INFORMATIQUE MULTI-ÉTIQUETTES MULTI-CLASSES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **14.09.2022 IN 202221052587**

(43) Date of publication of application:
**20.03.2024 Bulletin 2024/12**

(73) Proprietor: **Tata Consultancy Services Limited Maharashtra (IN)**

(72) Inventors:
• **UKIL, ARIJIT**
**700160 Kolkata, West Bengal (IN)**
• **DEB, TRISROTA**
**700160 Kolkata, West Bengal (IN)**
• **SAHU, ISHAN**
**700160 Kolkata, West Bengal (IN)**
• **RACHA, SAI CHANDER**
**500081 Hyderabad, Telangana (IN)**
• **KHANDELWAL, SUNDEEP**
**201309 Noida, Uttar Pradesh (IN)**
• **PAL, ARPAN**
**700160 Kolkata, West Bengal (IN)**
• **GARAIN, UTPAL**
**700108 Kolkata, West Bengal (IN)**
• **SAHA, SOUMADEEP**
**700108 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **CAI WENJIE ET AL: "Classification of multi-lead ECG with deep residual convolutional neural networks", 20220718, vol. 43, no. 7, 18 July 2022 (2022-07-18), XP020428386, DOI: 10.1088/1361-6579/AC7939**
• **JIMÉNEZ-SERRANO SANTIAGO ET AL: "From 12 to 1 ECG lead: multiple cardiac condition detection mixing a hybrid machine learning approach with a one-versus-rest classification strategy", 20220628, vol. 43, no. 6, 28 June 2022 (2022-06-28), XP020426911, DOI: 10.1088/1361-6579/AC72F5**
• **XU ZHUOYANG ET AL: "Abnormality classification from electrocardiograms with various lead combinations", 20220718, vol. 43, no. 7, 18 July 2022 (2022-07-18), XP020464753, DOI: 10.1088/1361-6579/AC70A4**
• **REYNA MATTHEW A ET AL: "Issues in the automated classification of multilead ecgs using heterogeneous labels and populations", 20220826, vol. 43, no. 8, 26 August 2022 (2022-08-26), XP020430850, DOI: 10.1088/1361-6579/AC79FD**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 339 811 B1

- YURKOV E F ET AL: "Diagnostic Model that Takes Medical Preferences into Account. Prediction of the Clinical Status of Prostate Cancer", JOURNAL OF COMMUNICATIONS TECHNOLOGY AND ELECTRONICS, NAUKA/ INTERPERIODICA PUBLISHING, MOSCOW, RU, vol. 64, no. 8, 19 August 2019 (2019-08-19), pages 834 - 845, XP036866385, ISSN: 1064-2269, [retrieved on 20190819], DOI: 10.1134/ S1064226919080266

**Description**

CROSS-REFERENCE TO RELATED APPLICATIONS AND PRIORITY

The present application claims priority to Indian application no. 202221052587, filed on September 14, 2022.

TECHNICAL FIELD

The present invention generally relates to the field of clinical model evaluation and, more particularly, to a method and system for evaluating clinical efficacy of multi-label multi-class computational diagnostic models.

BACKGROUND

Machine learning techniques have been applied to a wide set of diagnostic problems (for example, diagnosis of diabetic complications, Arrhythmia detection and classification etc.), which are often multi-label, i.e. where one or more diagnosis are detected from one diagnostic sample. Evaluation of such computational diagnostic models is often done using metrics which are used for evaluating conventional machine learning models. This however poses a challenge as models evaluated on different sets of metrics cannot be compared. Further, the choice of metric can serve to highlight key strengths of a model and ignore its weaknesses. Different metrics do not agree on comparative performance of models either, thus the choice of best diagnostic model can be dictated by choice of metric.

Additionally, results reported on several metrics are not necessarily informative enough from a clinical perspective. A large set of scores, measuring different aspects of performance does not help in determining the model that is better for clinical applications. Since the metrics are borrowed from machine learning, where requirements are different, a higher score on a certain metric does not necessarily translate to better diagnostic performance, and vice versa. Unlike problems in machine learning where requirements are varied, in clinical practice some facts are ubiquitous, and can be treated like gospel. For instance, a wrong diagnosis is worse than a missed diagnosis which is in turn worse than over diagnosis up to a certain extent. The standard metrics used in a multi-label setting (Hamming Loss, subset accuracy, etc.) does not reflect this. There might also be a scenario where certain sets of diagnosis have similar treatment plans and outcomes, thus making certain types of missed diagnosis less deleterious. The principle of risk avoidance states that in a computational diagnostic model, sensitivity should be correlated to cost (or lethality) with significant ailments having markedly higher sensitivity than minor issues. However, when this comes at a cost of specificity, it might lead to alarm fatigue. Thus, a conventional multi-label metric does not align with the highly context dependent clinical principles and practice when rating a diagnostic model and is unable to capture the critically important features that ought to be present in a diagnostic model.

CAI WENUJIE ET AL: "Classification of multi-lead ECG with deep residual convolutional neural networks" discloses automatic electrocardiogram (ECG) interpretation based on deep learning methods is attracting increasing attention. **In** this study, propose a novel method to accurately classify multi-lead ECGs using deep residual neural networks. Approach. ECG recordings from seven different open databases were provided by PhysioNet/Computing in Cardiology Challenge 2021. All the ECGs were pre-processed to obtain the same sampling rate. The label inconsistency among the databases was corrected by adding or removing specific labels. A label mask was created to filter out potentially incorrectly labelled data. Five models based on deep residual convolutional neural networks were optimized using an asymmetric loss function to classify multi-lead ECGs. Main results. The proposed method achieved an official challenge score of 0.54, 0.52, 0.50, 0.51, and 0.50 on twelve-lead, six-lead, four-lead, three-lead, and two-lead ECG test sets, respectively. These scores were ranked 5th, 3rd, 7th, 5th and 7th, respectively, in the challenge. Significance. The proposed method can correct the differential labeling tendency of databases from different sources and exhibits good generalization for classifying multi-lead ECGs in the hidden test set. The proposed models have the potential for clinical applications.

JIMENEZ-SERRANO SANTIAGO ET AL: "From 12 to 1 ECG lead: multiple cardiac condition detection mixing a hybrid machine learning approach with a one-versus-rest classification strategy" discloses detecting different cardiac diseases using a single or reduced number of leads is still challenging. This work aims to provide and validate an automated method able to classify ECG and DOI. recordings. Performance using complete 12-lead systems, reduced lead sets, and single-lead ECGs is evaluated and compared. Approach. Seven different databases with 12-lead ECGs were provided during the PhysioNet/Computing in Cardiology Challenge 2021, where 88 253 annotated samples associated with none, one, or several cardiac conditions among 26 different classes were released for training, whereas 42 896 hidden samples were used for testing. After signal preprocessing, 81 features per ECG-lead were extracted, mainly based on heart rate variability, QRST patterns and spectral domain. Next, a One-versus-Rest classification approach made of independent binary classifiers for each cardiac condition was trained. This strategy allowed each ECG to be classified as belonging to none, one or several classes. For each class, a classification model among two binary supervised classifiers and one hybrid unsupervised-supervised classification system was selected. Finally, we performed a 3-fold cross-validation to assess the system's performance. Main results. Our classifiers received scores of 0.39, 0.38, 0.39, 0.38, and 0.37 for the 12, 6, 4, 3 and 2-lead versions of the hidden test set with the Challenge evaluation metric (CM). Also, obtained a mean G-score of 0.80, 0.78, 0.79, 0.79, 0.77 and 0.74 for the 12, 6, 4, 3, 2 and 1-lead subsets with the public training set during our 3-fold cross-validation. Significance. We proposed and tested a machine learning approach focused on flexibility for identifying multiple cardiac conditions using one or more ECG leads. Our minimal-lead approach may be beneficial for novel portable or wearable ECG devices used as screening tools, as it can also detect multiple and concurrent cardiac conditions.

XU ZHUOYANG ET AL: "Abnormality classification from electrocardiograms with various lead combinations" discloses as cardiovascular diseases are a leading cause of death, early and accurate diagnosis of cardiac abnormalities for a lower cost becomes particularly important. Given electrocardiogram and DOI. (ECG) datasets from multiple sources, there exist many challenges to the development of generalized models that can identify multiple types of cardiac abnormalities from both 12-lead ECG signals and reduced-lead ECG signals. In this study, our objective is to build robust models that can accurately classify 30 types of abnormalities from various lead combinations of ECG signals. Approach. Given the challenges of this problem, we propose a framework for building robust models for ECG signal classification. Firstly, a preprocessing workflow is adopted for each ECG dataset to mitigate the problem of data divergence. Secondly, to capture the lead-wise relations, we use a squeeze-and excitation deep residual network as our base model. Thirdly, we propose a cross-relabeling strategy and apply the sign-augmented loss function to tackle the corrupted labels in the data. Furthermore, we utilize a pos-if-any-pos ensemble strategy and a dataset-wise cross-evaluation strategy to handle the uncertainty of the data distribution in the application. Main results. In the Physionet/-Computing in Cardiology Challenge 2021, our approach achieved the challenge metric scores of 0.57, 0.59, 0.59, 0.58, 0.57 on 12-, 6-, 4-, 3- and 2-lead versions and an averaged challenge metric score of 0.58 over all the lead versions. Significance. Using the proposed framework, we have developed the models from several large datasets with sufficiently labeled abnormalities. Our models are able to identify 30 ECG abnormalities accurately based on various lead combinations of ECG signals. The performance on hidden test data demonstrates the effectiveness of the proposed approaches.

REYNA MATTHEW A ET AL: "Issues in the automated classification of multilead ecgs using heterogeneous labels and populations" discloses the standard twelve-lead electrocardiogram (ECG) is a widely used tool for monitoring cardiac function and diagnosing cardiac disorders. The development of smaller, lower-cost, and easier-to-use ECG devices may improve access to cardiac care in lower-resource environments, but the diagnostic potential of these devices is unclear. This work explores these issues through a public competition: the 2021 PhysioNet Challenge. In addition, we explore the potential for performance boosting through a meta-learning approach. Approach. We sourced 131,149 twelve-lead ECG recordings from ten international sources. We posted 88,253 annotated recordings as public training data and withheld the remaining recordings as hidden validation and test data. We challenged teams to submit containerized, open-source algorithms for diagnosing cardiac abnormalities using various ECG lead combinations, including the code for training their algorithms. We designed and scored the algorithms using an evaluation metric that captures the risks of different misdiagnoses for 30 conditions. After the Challenge, we implemented a semi-consensus voting model on all working algorithms. Main results. A total of 68 teams submitted 1,056 algorithms during the Challenge, providing a variety of automated approaches from both academia and industry. The performance differences across the different lead combinations were smaller than the performance differences across the different test databases, showing that generalizability posed a larger challenge to the algorithms than the choice of ECG leads. A voting model improved performance by 3.5%. Significance. The use of different ECG lead combinations allowed us to assess the diagnostic potential of reduced-lead ECG recordings, and the use of different data sources allowed us to assess the generalizability of the algorithms to diverse institutions and populations. The submission of working, open-source code for both training and testing and the use of a novel evaluation metric improved the reproducibility, generalizability, and applicability of the research conducted during the challenge.

SUMMARY

Embodiments of the present disclosure present technological improvements as solutions to one or more of the above-mentioned technical problems recognized by the inventors in conventional systems. The invention is disclosed in the appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for evaluating clinical efficacy of multi-label multi-class computational diagnostic models, according to some embodiments of the present disclosure.

FIGS. 2A and 2B, collectively referred as FIG. 2, are flow diagrams illustrating method for evaluating clinical efficacy of multi-label multi-class computational diagnostic models, according to some embodiments of the present disclosure.

FIG. 3 is an alternative representation of flow diagram of FIG. 2, according to some embodiments of the present disclosure.

FIG. 4 is a graph illustrating results of Challenge Metric.

FIG. 5 illustrates a comparison of result of an ideal metric with a plurality of conventional metrics.

FIGS. 6 and 7 illustrate comparison of values calculated by method illustrated in FIG. 2 with a plurality of metrics including accuracy, subset accuracy, hamming loss, F1 score and challenge metric for diagnosis predicted for different diagnostic samples, according to some embodiments of the present disclosure.

FIGS. 8 and 9 illustrate effect of relative prevalence of a diagnostic condition on method illustrated in FIG. 2 and a plurality of metrics including accuracy, subset accuracy, hamming loss, F1 score and challenge metric, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

With machine learning based approaches showing promise in the multi-label multi-class paradigm, they are being widely adopted to computational diagnostic models. When evaluating these models, several factors prove to be important, like sensitivity, specificity, risk avoidance, etc. Existing metrics are usually borrowed from machine learning, and since each metric is usually designed to pick up on certain features, the current consensus is to report results on a large set of metrics. The choice of metrics can serve to downplay limitations of a model, and different choice of metrics can change the order relation amongst several competing models. It is challenging to compare efficacy of models which have been evaluated on different sets of metrics, and even if that is not the case, it is not clear how to summarize information from several metrics to choose a clinically applicable diagnostic model. From a diagnostic standpoint, the metrics themselves are far from perfect, often biased by prevalence of negative samples or other statistical factors.

Often, the multi-label multi-class computational diagnostic models are classifiers implemented using machine learning techniques. To evaluate the quality of a classifier $f_\theta$ on a dataset $\mathcal{D}$ comprising a plurality of diagnostic samples, it is sufficient to analyze a set $\mathcal{P} = \{(\hat{x}_i, y_i) | \forall i \text{ such that } (z_i, y_i) \in \mathcal{D}\}$, wherein $\hat{x}_i$ is prediction of the classifier for a diagnostic sample $z_i$ which has a ground truth label $y_i$ in the dataset $\mathcal{D}$. The job of a metric, given such a set $\mathcal{P}$ is to provide a number or a score which is correlated to the performance of the classifier.

State of the art metrics that are suitable for such an evaluation are bipartition based metrics which are again broadly divided into two categories: label based (listed in Table 1) and example based (listed in Table 2). The example based metrics assign a score based on averages over certain functions of the actual and predicted label sets. Label based metrics on the other hand compute the prediction performance of each label in isolation and then compute averages over labels. Certain other binary metrics have been proposed in a clinical diagnostic context, like threat score (Hicks SA et al. On evaluation metrics for medical applications of artificial intelligence. Scientific Reports. 2022;5979. doi:10.1038/s41598-022-09954-8.) or Mathews Correlation Coefficient, however they are not generally used in a multi-label context.

Table 1

| Metric | Definition |
|---|---|
| Macro-precision | $\dfrac{1}{P} \displaystyle\sum_{j=1}^{P} \dfrac{tp_j}{tp_j + fp_j}$ |

(continued)

| Metric | Definition |
|---|---|
| Macro-recall | $$\frac{1}{P}\sum_{j=1}^{P}\frac{tp_j}{tp_j+fn_j}$$ |
| Macro-F1-score | $$\frac{1}{P}\sum_{j=1}^{P}F1_j, F1_j=\frac{2p_jr_j}{p_j+r_j}$$ |
| Micro-precision | $$\frac{\sum_{j=1}^{P}tp_j}{\sum_{j=1}^{P}tp_j+\sum_{j=1}^{P}fp_j}$$ |
| Micro-recall | $$\frac{\sum_{j=1}^{P}tp_j}{\sum_{j=1}^{P}tp_j+\sum_{j=1}^{P}fn_j}$$ |
| Micro-F1-score | $$\frac{2*micro-precision-micro-recall}{micro-precision+micro-recall}$$ |

Table 2

| Metric | Definition |
|---|---|
| Hamming loss | $$\frac{1}{N}\sum_{j=1}^{N}\frac{1}{P}|\hat{x}_i \Delta y_i|$$ |
| Accuracy | $$\frac{1}{N}\sum_{j=1}^{N}\frac{|\hat{x}_i \cap y_i|}{|\hat{x}_i \cup y_i|}$$ |
| Precision | $$\frac{1}{N}\sum_{j=1}^{N}\frac{|\hat{x}_i \cap y_i|}{|y_i|}$$ |
| Recall | $$\frac{1}{N}\sum_{j=1}^{N}\frac{|\hat{x}_i \cap y_i|}{|\hat{x}_i|}$$ |
| F1-score | $$\frac{1}{N}\sum_{j=1}^{N}\frac{2\times|\hat{x}_i \cap y_i|}{|\hat{x}_i|+|y_i|}$$ |
| Subset accuracy | $$\frac{1}{N}\sum_{j=1}^{N}I(\hat{x}_i = y_i)$$ |
| Challenge Metric (CM) (Alday EAP et al. Classification of 12-lead ECGs: the PhysioNet/ Computing in Cardiology Challenge 2020. Physiological Measurement. 2021;41(12):124003. doi:10.1088/1361-6579/abc960.) | $$a_{j,k}=\sum_{i=1}^{N}\frac{I(a_j \in \hat{x}_i \, and \, a_k \in y_i)}{|\hat{x}_i \cup y_i|}$$ $$s_{unnorm}=\sum_{k=1}^{P}\sum_{j=1}^{P}a_{jk}w_{jk}$$ $$CM=\frac{s_{unnorm}-s_{inactive}}{s_{perfect}-s_{inactive}}$$ |

Label based metrics in use today take the form of micro or macro averages of binary classification metrics (given in Table 1), such as precision, recall and $F_1$ (or the general $F_\beta$) to provide summary information of performance across several categories. Specificity is unsuited in the clinical domain, due to the class imbalance usually present in diagnostic datasets, where negative examples are plentiful. A macro averaged measure is computed by first independently computing the binary metric for each class and then averaging over them. A micro average on the other hand will aggregate the statistics across classes and compute the final metric. However, both of these approaches have their own drawbacks. The micro average favors classifiers with stronger performance on predominant classes whereas the macro average favors classifiers suited to detecting rarely occurring classes. In a clinical setting where it is very common for certain presentations to be very rare, the micro average measures are less meaningful, as it is the rare diseases that are often of most concern and would benefit greatly from intervention. From a machine learning point of view, it is unreasonable to expect a classifier to have a high sensitivity when it is only provided a few examples, additionally if a diagnostic criteria is indeed extremely rare, its influence on the quality of the diagnostic system should be limited.

Example based metrics (given in Table 2) are specifically designed to pick out certain key features of a multi-label classifier. It is in general inadequate to compute just one or two metrics, as they each have individual properties which provide beneficial cues. One notable recent work by Alday, et. al (Classification of 12-lead ECGs: the PhysioNet/ Computing in Cardiology Challenge 2020. Physiological Measurement. 2021;41(12):124003. doi:10.1088/1361-6579/abc960), set out to design a metric called Challenge Metric (CM) that takes clinical outcomes into account in a multi-class multi-label diagnostic setting. Here, initially a multi class confusion matrix $A = [a_{ij}]$ is defined according to equation 1. Next, a score $t(Y,X)$ is computed according to equation 2, wherein $w_{ij}$ is a weight matrix which assigns partial rewards to incorrect guesses. $w_{ii} = 1$ and in general $0 < w_{ij} \leq 1$. Final score is computed according to equation 3. This metric which is a weighted version of accuracy is limited to be used on the PhysioNet 2020/21 dataset, however with additional domain knowledge inputs, can be used in different contexts.

$$a_{ij} = \sum_{k=1}^{N} a_{ijk}, \text{ wherein } a_{ijk} = \begin{cases} \frac{1}{|\hat{x}_k \cup y_k|} & if \ c_i \in \hat{x}_k \ and \ c_j \in y_k \\ 0, & otherwise \end{cases} \tag{1}$$

$$t(Y, X) = \sum_i \sum_j w_{ij} a_{ij} \tag{2}$$

$$CM = \frac{t(Y,X) - t(Y, \{NSR\})}{t(Y,Y) - t(Y, \{NSR\})} \tag{3}$$

All of these metrics, however, do not adequately take clinical aspects into account, for example the fact that over-diagnosis is less harmful than missed-diagnosis, or the criticality of the diagnosis. Keeping the clinical considerations in mind, and in consultation with experts from the domain, inventors have outlined qualities (alternatively referred as clinical criteria) a clinically aligned metric should demonstrate. They are: (i) missed diagnosis is more harmful than over-diagnosis; (ii) wrong diagnosis is more harmful than over-diagnosis and missed diagnosis; (iii) some diagnosis have more clinical significance; (iv) some diagnosis are contradictory and should be disqualifying; and (v) quality of a diagnostic model should not depend on relative proportions of diseases present in the population (dataset distribution independence). Some of the existing metrics are analyzed with respect to these features. In particular, it is checked whether wrong diagnosis (WD) is more heavily penalized than missed diagnosis (MD) which in turn is penalized worse than over-diagnosis (OD), while the right diagnosis (PD) comes out on top i.e., $score_{WD} < score_{MD} < score_{OD} < score_{RD}$. For this analysis, four classifiers $\mathcal{P}_O$, $\mathcal{P}_M$, $\mathcal{P}_W$, and $\mathcal{P}$ which predict only over diagnosis, missed diagnosis, wrong diagnosis and right diagnosis respectively are considered.

Macro precision and macro recall metrics cannot be used in isolation. However, Macro F1 which is a macro average of the harmonic means of precision and recall is a serviceable metric. Macro F1 is defined according to equation 4, where $p_j$ and $r_j$ are precision and recall for $j^{th}$ disease class in the dataset. Considering a case where $r_j(\mathcal{P}_M) \geq p_j(\mathcal{P}_O)$, inequalities represented in equations 5, 6 and 7 can be derived. This is the exact opposite inequality that is desired if it holds for all j, and even if it is only true for some j, no guarantee can be made that a diagnostic model that always misses diagnoses is worse than the one that always gives over-diagnosis.

$$MacroF_1(\mathcal{P}) = \frac{1}{P} \sum_{j=1}^{P} F_{1(j)}(\mathcal{P}), \text{ wherein } F_{1(j)}(\mathcal{P}) = \frac{2 \cdot p_j(\mathcal{P}) \cdot r_j(\mathcal{P})}{p_j(\mathcal{P}) + r_j(\mathcal{P})} \tag{4}$$

$$\frac{2 \cdot r_j(\mathcal{P}_M)}{1+r_j(\mathcal{P}_M)} \geq \frac{2 \cdot p_j(\mathcal{P}_O)}{1+p_j(\mathcal{P}_O)} \tag{5}$$

$$\frac{2 \cdot p_j(\mathcal{P}_M) \cdot r_j(\mathcal{P}_M)}{p_j(\mathcal{P}_M)+r_j(\mathcal{P}_M)} \geq \frac{2 \cdot p_j(\mathcal{P}_O) \cdot r_j(\mathcal{P}_O)}{p_j(\mathcal{P}_O)+r_j(\mathcal{P}_O)} \tag{6}$$

$$F_{1(j)}(\mathcal{P}_M) \geq F_{1(j)}(\mathcal{P}_O) \tag{7}$$

Similar to their macro counterparts, micro precision and recall cannot be used in isolation, but micro $F_1$ can be used independently to evaluate the quality of a computational diagnostic model. micro $F_1$ is defined according to equation 8.

$$MicroF_1(\mathcal{P}) = \frac{2 \cdot micro-precision \cdot micro-recall}{micro-precision+micro-recall} \tag{8},$$

wherein micro-precision and micro-recall are defined in Table 1.

It is known that $fp_j = 0$ in $\mathcal{P}_M$ and $fn_j = 0$ in $\mathcal{P}_O$. So, micro-precision of $\mathcal{P}_M$ is 1 and $MicroF_1(\mathcal{P}_M)$ is calculated by equation 9. Similarly micro-recall of $\mathcal{P}_O$ is 1 and $MicroF_1(\mathcal{P}_O)$ is calculated by equation 10. Hence, $MicroF_1(\mathcal{P}_M) \geq MicroF_1(\mathcal{P}_O)$ whenever $micro$ - $recall(\mathcal{P}_M) \geq micro$ - $precision(\mathcal{P}_O)$. This means that if two diagnostic models have same number of true positives, one of them has higher number of false positives and the other one has higher number of false negatives, then, $MicroF_1(\mathcal{P}_M) \geq MicroF_1(\mathcal{P}_O)$. This is the opposite of desired ordering in clinical practice as false negatives are generally more deleterious.

$$MicroF_1(\mathcal{P}_M) = \frac{2 \cdot micro-recall(\mathcal{P}_M)}{1+micro-recall(\mathcal{P}_M)} \tag{9}$$

$$MicroF_1(\mathcal{P}_O) = \frac{2 \cdot micro-precision(\mathcal{P}_O)}{1+micro-precision(\mathcal{P}_O)} \tag{10}$$

Thus, it can be seen that label based metrics cannot be used for evaluating clinical diagnostic models. Now, example based metrics are analyzed by considering predictions $m_i$, $o_i$ and $w_i$ as missed diagnosis, over diagnosis and wrong diagnosis respectively for a ground truth label $y_i$. The first metric that is analyzed is hamming loss which is defined in Table 2. From the definition, equation 11 follows. So, missing k diagnoses is penalized just as harshly as producing k over-diagnoses. Since classifiers are tuned to target certain metrics, it must be noted that hamming loss is usually not optimal for sensitive systems.

$$hloss(\{(m_i, y_i)\}) = hloss(\{(o_i, y_i)\}) \ whenever \ |y_i - m_i| = |o_i - m_i| \tag{11}$$

The next metric that is analyzed is accuracy which is defined in Table 2. From the definition, equation 12 follows which in turn implies the inequality in equation 13. Thus, the inequality doesn't hold in general. Also, it is widely known that accuracy is an unreliable measure in a clinical context where imbalanced datasets are the norm.

$$|m_i| \cdot |o_i| \geq y_i{}^2 \tag{12}$$

$$accuracy(\{(m_i, y_i)\}) = accuracy(\{(o_i, y_i)\}) \tag{13}$$

Next, subset accuracy defined in Table 2 is analyzed. From the definition, equation 14 follows. It means that the subset accuracy metric gives same value for all types of diagnoses and hence cannot be used in a clinical setting.

$$SAccuracy(\{(m_i, y_i)\}) = SAccuracy(\{(w_i, y_i)\}) = SAccuracy(\{(o_i, y_i)\}) = 0 \tag{14}$$

Further, $F_1$ score defined in Table 2 is analyzed. Suppose $|y_i| = k$, $|m_i| = k - 1$ (one diagnosis is missed) and $|o_i| = k + r$ (r extra predictions), then, equation 15 can be derived. So, the inequality doesn't hold in all the scenarios. As in the case of label based metrics, example based precision and recall aren't meaningful in isolation, and aren't analyzed.

$$F_1(\{(m_i, y_i)\}) \geq F_1(\{(o_i, y_i)\}) \text{ whenever } r \geq \left\lceil \frac{k}{k-1} \right\rceil \tag{15}$$

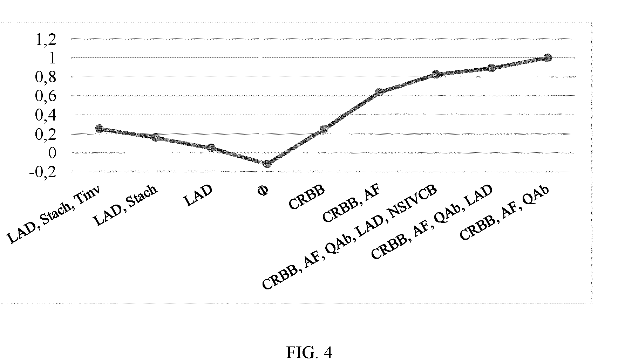

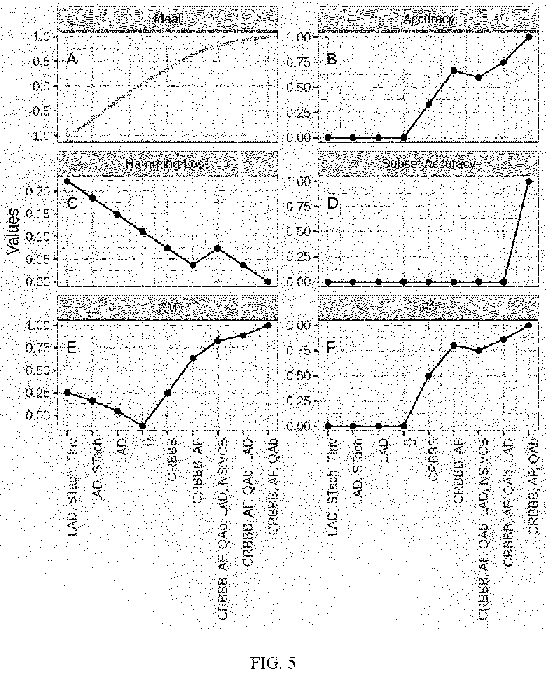

Next, the challenge metric defined in equation 3 is analyzed. Since $w_{ij}$ is integral to the metric, it is limited for use on the PhysioNet 2020/21 Dataset, which is a multi-label 12 lead ECG dataset with 27 cardio-vascular diagnostic conditions. Without the weight matrix this is the same as accuracy and inherits all its problems. Even on the PhysioNet 2020/21 dataset it does not guarantee satisfaction of the inequality. FIG. 4 illustrates results of challenge metric. It can be seen that the CM does not follow order relation between over, missed, and wrong diagnosis. For example, a completely wrong diagnosis {LAD, Stach, TInv} has a higher score than partially sensitive diagnosis {CRBBB}. The ground truth label is {CRBB, AF, QAb}. FIG. 5 illustrates a comparison of result of an ideal metric with a plurality of conventional metrics including accuracy, subset accuracy, hamming loss, CM and F1 score, according to some embodiments of the present disclosure. It can be seen that almost all of the metrics fail on the basic clinical criteria. The plurality of conventional metrics have other issues as well, for instance they are heavily dataset dependent, and straightforward comparison between two datasets with varying proportions of different labels is meaningless. Additionally, relative significance of different classes has not been addressed, keeping clinical practice in mind.

To overcome the above mentioned drawbacks of existing metrics, embodiments of present disclosure provide a new metric and a method of evaluating multi-label multi-class clinical diagnostic models which performs in accordance with the clinical criteria laid out and inculcates the clinically desirable properties. Initially a dataset comprising a plurality of diagnostic samples and corresponding ground truth is received. Further, a diagnosis corresponding to each of the plurality of diagnostic samples is predicted using a multi-label multi-class computational diagnostic model. Then, the predicted diagnosis is classified as (i) a wrong diagnosis, (ii) a missed diagnosis, (iii) an over diagnosis or (iv) a right diagnosis, based on which a first penalty is calculated. Further, a second penalty is calculated for each diagnostic sample based on a pre-defined contradiction matrix. The first penalty and second penalty are summed up to compute a pre-score for each diagnostic sample. Further, a total score for the multi-label multi-class computational diagnostic model is calculated as sum of the pre-scores of each diagnostic sample. Finally, the multi-label multi-class computational diagnostic model is evaluated using a metric that is based on (i) the total score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only.

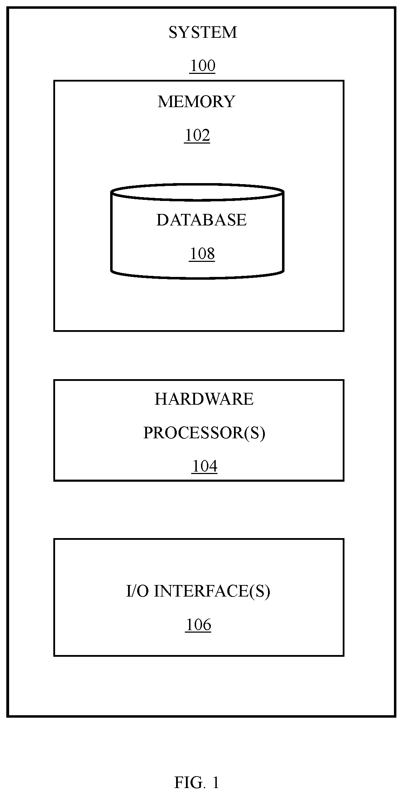

Referring now to the drawings, and more particularly to FIGS. 1 to 3 and FIGS. 6 to 9 where similar reference characters denote corresponding features consistently throughout the figures, there are shown preferred embodiments and these embodiments are described in the context of the following exemplary system and/or method.

FIG. 1 illustrates an exemplary block diagram of a system for evaluating clinical efficacy of multi-label multi-class computational diagnostic models, according to some embodiments of the present disclosure. In an embodiment, the system 100 includes one or more processors 104, communication interface device(s) 106 or Input/Output (I/O) interface(s) 106 or user interface 106, and one or more data storage devices or memory 102 operatively coupled to the one or more processors 104. The memory 102 comprises a database 108. The one or more processors 104 that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) 106 receives a dataset comprising a plurality of diagnostic samples and their corresponding ground truth as input and gives score of the multi-label multi-class computational diagnostic model as output.

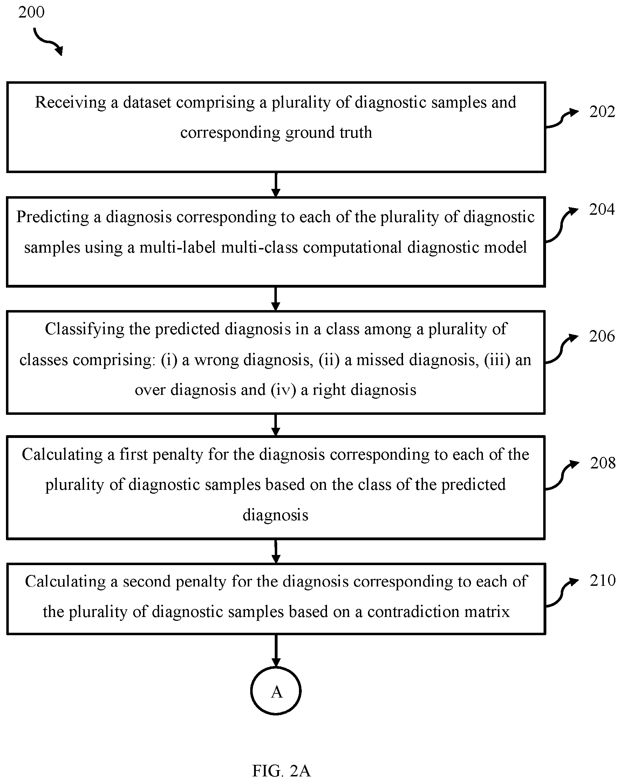

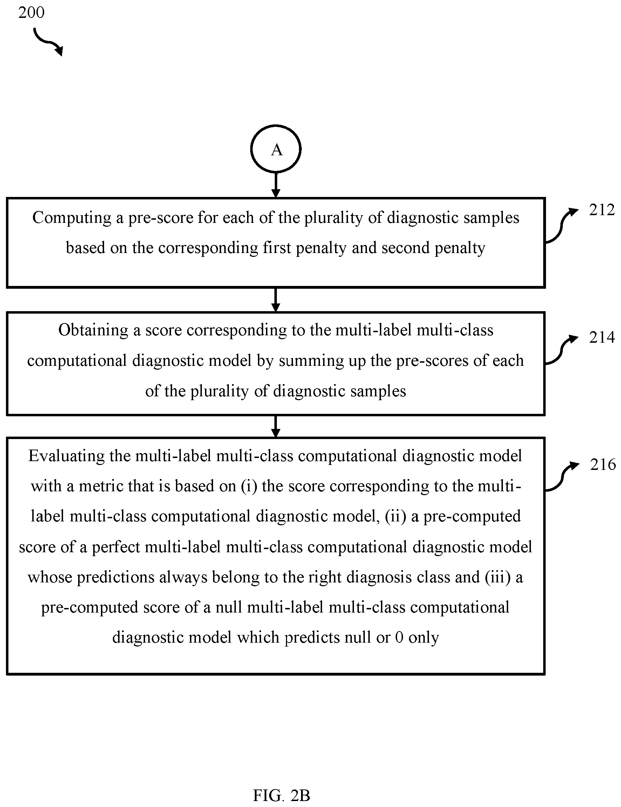

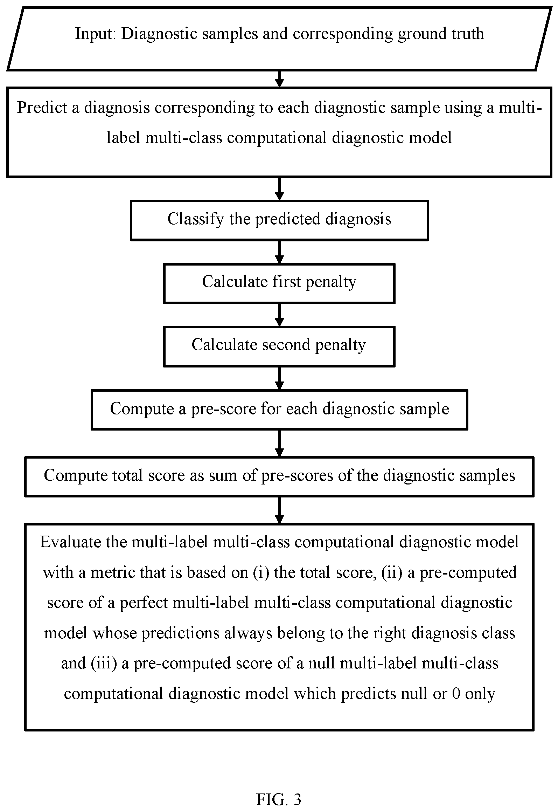

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. Functions of the components of system 100 are explained in conjunction with flow diagram depicted in FIGS. 2 and 3 for evaluating clinical efficacy of multi-label multi-class computational diagnostic models.

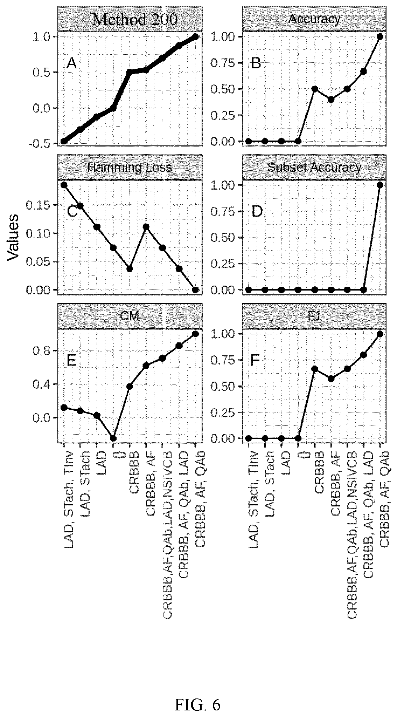

In an embodiment, the system 100 comprises one or more data storage devices or the memory 102 operatively coupled to the processor(s) 104 and is configured to store instructions for execution of steps of the method (200) depicted in FIGS. 2 and 3 by the processor(s) or one or more hardware processors 104. The steps of the method of the present disclosure will now be explained with reference to the components or blocks of the system 100 as depicted in FIG. 1, the steps of flow diagram as depicted in FIGS. 2 and 3, and the experimental results illustrated in FIGS. 6 to 9. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

FIG. 2 is a flow diagram illustrating method 200 for evaluating clinical efficacy of multi-label multi-class computational diagnostic models, according to some embodiments of the present disclosure. FIG. 3 is an alternative representation of the process flow of the method 200 illustrated in FIG. 2. At step 202 of the method 200, the one or more hardware processors 104 are configured to receive a dataset comprising a plurality of diagnostic samples and corresponding ground truth (alternatively referred as label). The dataset can be mathematically represented as $\mathcal{D} = \{(z_i, y_i) | i \in 1, 2, \ldots N\}$ wherein $z_i$ and $y_i$ are $i^{th}$ diagnostic sample and ground truth respectively. Each $y_i$ is a set of diagnoses (alternatively referred as diagnostic conditions) which is drawn from a fixed set of possible diagnostic conditions $A = \{a_1, a_2, \ldots, a_P\}$.

Once the dataset is received, a diagnosis corresponding to each of the plurality of diagnostic samples is predicted using a multi-label multi-class computational diagnostic model at step 204 of the method 200. The predicted diagnosis comprises one or more diagnostic conditions. In an embodiment, the multi-label multi-class computational diagnostic model is a classifier $f_\theta : \mathbb{R}^d \to 2^A$ $(z_i \in \mathbb{R}^d)$. Given a diagnostic sample $z_i$ the classifier predicts the corresponding label $x_i = f_\theta(z_i)$ which is approximately the ground truth $y_i$ for some (potentially hidden) parameters $\theta$ of the classifier. In another embodiment, output of the classifier is in the form of scores which are correlated to probabilities of a certain diagnostic condition being present, i.e. $g_\theta : \mathbb{R}^d \to [0,1]^P$ and a thresholding protocol $t(z_i) \mapsto t_i \in [0,1]^P$. A successful classifier should satisfy the condition given in equation 16. It means that score of the classifier corresponding to a diagnostic sample $z_i$ is greater when it predicts a diagnostic condition $a_j$ which is an element of the ground truth $y_i$ than when it predicts a diagnostic condition $a_k$ which is not an element of the ground truth $y_i$. In other words, score is higher when the prediction is closer to the ground truth.

$$g(z_i)_j > g(z_i)_k \text{ if } a_j \in y_i \text{ and } a_k \notin y_i \tag{16}$$

The predicted diagnosis is a set of all diagnostic conditions that satisfy a prediction threshold as given in equation 17.

$$\hat{x}_i = \{a_j | x_{ij} = 1, \forall j \in \{1, 2, \ldots P\}, \text{ where } x_{ij} = g_\theta(z_i)_j = \begin{cases} 1, if\ g_\theta(z_i)_j > t_{ij} \\ 0, otherwise \end{cases} \tag{17}$$

In an embodiment, the predicted diagnosis maybe processed before proceeding to step 206 of the method 200 by collapsing diagnostic conditions that are equivalent. For example, Premature Atrial Contraction (PAC) and Supraventricular Premature Beats (SVPB) are equivalent diagnostic conditions. So, any one of them will be retained among the set of all diagnostic conditions in the predicted diagnosis. At step 206 of the method 200, the predicted diagnosis is classified in a class among a plurality of classes comprising: (i) a wrong diagnosis, (ii) a missed diagnosis, (iii) an over diagnosis and (iv) a right diagnosis. The predicted diagnosis for a diagnostic sample from among the plurality of diagnostic samples is classified as (i) the wrong diagnosis if the predicted diagnosis and ground truth corresponding to the diagnostic sample are disjoint, i.e. $\hat{x}_i \cap y_i = \varnothing$, (ii) the missed diagnosis if the predicted diagnosis is a proper subset of the ground truth corresponding to the diagnostic sample, i.e. $\hat{x}_i \subset y_i$, (iii) the over diagnosis if the ground truth corresponding to the diagnostic sample is a proper subset of the predicted diagnosis i.e. $y_i \subset \hat{x}_i$, or (iv) the right diagnosis otherwise. Hence, for a predicted diagnosis $\hat{x}_i$ of a diagnostic sample $z_i$ having ground truth $y_i$, the sets $\hat{x}_i \cap y_i$, $y_i - \hat{x}_i$, and $\hat{x}_i - y_i$ correspond to right diagnosis, missed diagnosis and over diagnosis respectively. If $\hat{x}_i \cap y_i = \varnothing$, i.e., there are no diagnostic conditions common between the predicted diagnosis and the ground truth, then the predicted diagnosis is a wrong diagnosis.

Once the predicted diagnosis is classified, at step 208 of the method 200, a first penalty (mathematically represented as $a_{k(i)}$ for a diagnostic sample (k) is calculated for the diagnosis corresponding to each of the plurality of diagnostic samples based on the class of the predicted diagnosis. For example, the first penalty is calculated by one of: (i) $\frac{s_i}{n_i}$ if the predicted diagnosis is a right diagnosis, (ii) $\frac{-s_i}{n_i}$ if the predicted diagnosis is a missed diagnosis, (iii)

$$\frac{s_i}{n^*}\left[\frac{1}{|y_k|}\left(\sum_{c_j \in y_k} w_{i,j}\right) - 1\right]$$ if the predicted diagnosis is an over diagnosis, and (iv) 0 if the predicted diagnosis is a wrong diagnosis, wherein $s_i$ is pre-defined significance weight corresponding to class of diagnostic conditions in the dataset. This reflects the fact that all diagnostic conditions might not be equally relevant, and classes which are critical have a higher value of $s_i$, so their contribution to the final score is larger. The significance weights can be set to 1 for all the diagnostic conditions if their relative importance is the same. $n_i$ is number of occurrences of the predicted diagnosis in the dataset and is introduced in the first penalty to ensure that prevalence of diagnostic conditions doesn't affect the final score. n* is calculated by equation 18. $y_k$ is the ground truth corresponding to the diagnostic sample for which prediction is done. $w_{i,j}$ is a weight matrix comprising cost of misclassification as in Aldey et. al. (Classification of 12-lead ECGs: the PhysioNet/Computing in Cardiology Challenge 2020. Physiological Measurement. 2021;41(12):124003. doi:10.1088/1361-6579/abc960). This gives partial rewards to over diagnosis which are of similar nature in outcomes or treatment of the diagnostic conditions. If such a matrix is unavailable or not required, $w_{i,j}$ can be set to a constant value among (0, 1). By calculating the first penalty in this way, strict monotonicity of the first penalty is maintained by assigning highest first penalty to wrong diagnosis followed by missed diagnosis and over diagnosis thereby imbibing risk aversion principle of clinical diagnosis.

$$n^* = max\{n_i | \forall c_i \in y_k\} \qquad (18)$$

Once the first penalty for each diagnostic sample is calculated, at step 210 of the method 200, a second penalty for the diagnosis corresponding to each of the plurality of diagnostic samples is calculated based on a contradiction matrix which provides contradictory and non-contradictory pairs of diagnostic conditions. For example, hypotension and hypertension cannot occur together hence they are contradictory pairs. In an embodiment, the contradiction matrix is developed with the help of an expert in medical field. It can be mathematically represented as $C_{i,j}$ such that $C_{i,j}=1$ if diagnostic conditions $c_i$ and $c_j$ cannot occur together. The second penalty is calculated by equation 19 if $c_i$ is a diagnostic condition in the predicted diagnosis or 0 otherwise, wherein $n_i$ is number of occurrences of the predicted diagnosis in the dataset, $\hat{x}_k$ is the predicted diagnosis, and $s_j$ is pre-defined significance weight corresponding to the class of diagnosis.

$$\frac{-1}{n_i}\sum_{\forall j \ s.t. \ c_j \in \hat{x}_k} s_j \cdot C_{ij} \qquad (19)$$

The contradiction matrix is responsible for penalizing impossible or mutually exclusive diagnostic conditions. This ensures that the predictions are not only accurate but are logically consistent. This contradiction matrix reduces the space of possible predictions to exclude impossible diagnosis, for example, atrial fibrillation is marked by a lack of a P-wave in the ECG signal and sinus rhythms have a P-wave, thus precluding each other.

Once the second penalty is calculated, at step 212 of the method 200, a pre-score ($t_k$) for each of the plurality of diagnostic samples is computed based on the corresponding first penalty ($a_{k(i)}$) and second penalty ($b_{k(i)}$) as given in equation 20. Further, at step 214 of the method 200, a total score corresponding to the multi-label multi-class computational diagnostic model is obtained by summing up the pre-score of each of the plurality of diagnostic samples as given in equation 21, wherein Y = {$y_i | \forall i \in \{1,2, ... N\}$} and X = {$\hat{x}_i | \forall i \in \{1,2,... N\}$}. In other words, Y is set of ground truth labels in the dataset and X is set of predicted diagnosis for the diagnostic samples in the dataset.

$$t_k = \sum_{i=1}^{P} a_{k(i)} + b_{k(i)} \qquad (20)$$

$$t(Y, X) = \sum_{k=1}^{N} t_k \qquad (21)$$

Once the total score corresponding to the multi-label multi-class computational diagnostic model is obtained, at step 214 of the method 200, the multi-label multi-class computational diagnostic model is evaluated with a metric that is based on (i) the score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only. The metric $M_{CS}$ is given by equation 22, wherein $t(Y, Y)$ is the pre-computed score of a perfect multi-label multi-class computational diagnostic model and t(Y, $\varnothing$) is the pre-computed score of a null multi-label multi-class computational diagnostic model. In an embodiment, $t(Y, Y)$ and $t(Y, \varnothing)$ are computed using the steps 208 to 214 of the method 200 by assuming that the predicted diagnosis is always right diagnosis, and the predicted diagnosis is null respectively. This way of evaluating the multi-label multi-class computational diagnostic model using the metric (given by equation 22) ensures that a perfect model gets a maximum possible score of 1 and an inactive model that predicts nothing gets a score of 0.

$$M_{CS} = \frac{t(Y,X) - t(Y,\emptyset)}{t(Y,Y) - t(Y,\emptyset)} \tag{22}$$

USE CASE EXAMPLE AND EXPERIMENTAL RESULTS

As a use case example, the method 200 is applied on the PhysioNet 2020/21 challenge dataset, where 27 cardiovascular diseases (CVDs) are to be detected from 12 lead ECG signals. The classes of diagnostic conditions present are given in Table 3 with their corresponding significance weights. Table 4 illustrates the weight matrix. The contradiction matrix is defined with the help of domain experts. It is a square matrix of the diagnostic conditions listed in Table 3 and an entry in a cell of the matrix is 1 if diagnostic conditions corresponding to its row and column do not occur together.

Table 3

| Class of diagnostic condition | Acronym | Significance weight ($s_i$) |
|---|---|---|
| 1st Degree AV Block | IAVB | 0.5 (Critical) |
| Atrial Fibrillation | AF | 1 (Super critical) |
| Atrial Flutter | AFL | 1 (Super critical) |
| Bradycardia | Brady | 0.5 (Critical) |
| Complete Right Bundle Branch Block | CRBBB | 1 (Super critical) |
| Incomplete Right Bundle Branch Block | IRBBB | 0.25 |
| Left Anterior Fascicular Block | LAnFB | 0.5 (Critical) |
| Left Axis Deviation | LAD | 0.25 |
| Left Bundle Branch Block | LBBB | 1 (Super critical) |
| Low QRS Voltage | LQRSV | 1 (Super critical) |
| Nonspecific Intraventricular Conduction Disorder | NSIVCB | 0.25 |
| Pacing Rhythm | PR | 0.25 |
| Premature Atrial Contraction | PAC | 0.25 |
| Premature Ventricular Contractions | PVC | 0.25 |
| Prolonged PR Interval | LPR | 0.25 |
| Prolonged QT Interval | LQT | 0.5 (Critical) |
| Q Wave abnormal | QAb | 0.5 (Critical) |
| Right Axis Deviation | RAD | 0.25 |
| Right Bundle Branch Block | RBBB | 1 (Super critical) |
| Sinus Arrhythmia | SA | 0.5 (Critical) |
| Sinus Bradycardia | SB | 0.5 (Critical) |
| Normal Sinus Rhythm | NSR | 0.25 |
| Sinus Tachycardia | STach | 0.25 |
| Supraventricular Premature Beats | SVPB | 0.25 |
| T Wave Abnormal | Tab | 1 (Super critical) |
| T Wave Inversion | TInv | 0.25 |
| Ventricular Premature Beats | VPB | 0.25 |

Table 4

| | IAVB | AF | AFL | Brady | CRBBB | IRBBB | LAnFB | LAD | LBBB | LQRSV | NSIVCB | PR | PAC | PVC | LPR | LQT | QAb | RAD | RBBB | SA | SB | NSR | STach | SVPB | TAb | TInv | VPB |
|---|---|---|---|---|---|---|---|---|---|---|---|---|---|---|---|---|---|---|---|---|---|---|---|---|---|---|---|
| IAVB | 0.1 | 0.3 | 0.3 | 0.5 | 0.4 | 0.5 | 0.5 | 0.5 | 0.3 | 0.4 | 0.5 | 0.4 | 0.5 | 0.4 | 0.5 | 0.3 | 0.2 | 0.5 | 0.4 | 0.5 | 0.5 | 0.5 | 0.4 | 0.5 | 0.3 | 0.3 | 0.4 |
| AF | 0.3 | 0.1 | 0.5 | 0.3 | 0.4 | 0.3 | 0.3 | 0.3 | 0.5 | 0.4 | 0.3 | 0.4 | 0.3 | 0.4 | 0.3 | 0.5 | 0.4 | 0.3 | 0.4 | 0.3 | 0.3 | 0.2 | 0.4 | 0.3 | 0.5 | 0.5 | 0.4 |
| AFL | 0.3 | 0.5 | 0.1 | 0.3 | 0.4 | 0.3 | 0.3 | 0.3 | 0.5 | 0.4 | 0.3 | 0.4 | 0.3 | 0.4 | 0.3 | 0.5 | 0.4 | 0.3 | 0.4 | 0.3 | 0.3 | 0.2 | 0.4 | 0.3 | 0.5 | 0.5 | 0.4 |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| Brady | 0.5 | 0.3 | 0.3 | | 0.1 | 0.4 | 0.5 | 0.5 | 0.5 | 0.3 | 0.4 | 0.5 | 0.4 | 0.5 | 0.4 | 0.5 | 0.3 | 0.2 | 0.5 | 0.4 | 0.5 | 0.5 | 0.5 | 0.4 | 0.5 | 0.3 | 0.3 | 0.4 |
| CRBBB | 0.4 | 0.4 | 0.4 | 0.4 | | 0.1 | 0.4 | 0.5 | 0.5 | 0.4 | 0.5 | 0.5 | 0.5 | 0.4 | 0.5 | 0.4 | 0.5 | 0.3 | 0.5 | 0.1 | 0.4 | 0.4 | 0.3 | 0.5 | 0.4 | 0.4 | 0.4 | 0.5 |
| IRBBB | 0.5 | 0.3 | 0.3 | 0.5 | 0.4 | | 0.1 | 0.5 | 0.5 | 0.3 | 0.4 | 0.5 | 0.4 | 0.5 | 0.4 | 0.5 | 0.3 | 0.2 | 0.5 | 0.4 | 0.5 | 0.5 | 0.5 | 0.4 | 0.5 | 0.3 | 0.3 | 0.4 |
| LAnFB | 0.5 | 0.3 | 0.3 | 0.5 | 0.5 | 0.5 | | 0.1 | 0.5 | 0.4 | 0.4 | 0.5 | 0.5 | 0.5 | 0.5 | 0.4 | 0.5 | 0.2 | 0.5 | 0.5 | 0.5 | 0.5 | 0.4 | 0.5 | 0.5 | 0.3 | 0.3 | 0.5 |
| LAD | 0.5 | 0.3 | 0.3 | 0.5 | 0.5 | 0.5 | 0.5 | | 0.1 | 0.4 | 0.4 | 0.5 | 0.5 | 0.5 | 0.5 | 0.4 | 0.5 | 0.2 | 0.5 | 0.5 | 0.5 | 0.5 | 0.4 | 0.5 | 0.5 | 0.3 | 0.3 | 0.5 |
| LBBB | 0.3 | 0.5 | 0.5 | 0.3 | 0.4 | 0.3 | 0.4 | 0.4 | | 0.1 | 0.5 | 0.4 | 0.4 | 0.4 | 0.4 | 0.3 | 0.5 | 0.4 | 0.4 | 0.4 | 0.3 | 0.3 | 0.3 | 0.4 | 0.4 | 0.5 | 0.5 | 0.4 |
| LQRSV | 0.4 | 0.4 | 0.4 | 0.4 | 0.5 | 0.4 | 0.4 | 0.4 | 0.5 | | 0.1 | 0.4 | 0.5 | 0.4 | 0.5 | 0.4 | 0.5 | 0.3 | 0.4 | 0.5 | 0.4 | 0.4 | 0.3 | 0.5 | 0.4 | 0.4 | 0.4 | 0.5 |
| NSIVCB | 0.5 | 0.3 | 0.3 | 0.5 | 0.5 | 0.5 | 0.5 | 0.5 | 0.4 | 0.4 | | 0.1 | 0.5 | 0.5 | 0.5 | 0.5 | 0.4 | 0.2 | 0.5 | 0.5 | 0.5 | 0.5 | 0.4 | 0.5 | 0.5 | 0.3 | 0.3 | 0.5 |

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| P R | 0.4 | 0.4 | 0.4 | 0.4 | 0.5 | 0.4 | 0.5 | 0.5 | 0.4 | 0.5 | 0.5 | | 0.1 | 0.5 | 0.5 | 0.4 | 0.4 | 0.3 | 0.5 | 0.5 | 0.4 | 0.4 | 0.4 | 0.5 | 0.5 | 0.4 | 0.4 | 0.5 |
| P A C | 0.5 | 0.3 | 0.3 | 0.5 | 0.4 | 0.5 | 0.5 | 0.5 | 0.4 | 0.4 | 0.5 | 0.5 | | 0.1 | 0.5 | 0.5 | 0.4 | 0.2 | 0.5 | 0.4 | 0.5 | 0.5 | 0.4 | 0.5 | 0.1 | 0.3 | 0.3 | 0.5 |
| P V C | 0.4 | 0.4 | 0.4 | 0.4 | 0.5 | 0.4 | 0.5 | 0.5 | 0.4 | 0.5 | 0.5 | 0.5 | 0.5 | | 0.1 | 0.4 | 0.4 | 0.3 | 0.5 | 0.5 | 0.4 | 0.4 | 0.4 | 0.5 | 0.5 | 0.4 | 0.4 | 0.1 |
| L P R | 0.5 | 0.3 | 0.3 | 0.5 | 0.4 | 0.5 | 0.5 | 0.5 | 0.3 | 0.4 | 0.5 | 0.4 | 0.5 | 0.4 | | 0.1 | 0.3 | 0.2 | 0.5 | 0.4 | 0.5 | 0.5 | 0.5 | 0.4 | 0.5 | 0.3 | 0.3 | 0.4 |
| L Q T | 0.3 | 0.5 | 0.5 | 0.3 | 0.5 | 0.3 | 0.4 | 0.4 | 0.5 | 0.5 | 0.4 | 0.4 | 0.4 | 0.4 | 0.3 | | 0.1 | 0.3 | 0.4 | 0.5 | 0.3 | 0.3 | 0.3 | 0.4 | 0.4 | 0.5 | 0.5 | 0.4 |
| Q A b | 0.2 | 0.4 | 0.4 | 0.2 | 0.3 | 0.2 | 0.2 | 0.2 | 0.4 | 0.3 | 0.2 | 0.3 | 0.2 | 0.3 | 0.2 | 0.3 | | 0.1 | 0.2 | 0.3 | 0.2 | 0.2 | 0.1 | 0.3 | 0.2 | 0.4 | 0.4 | 0.3 |
| R A D | 0.5 | 0.3 | 0.3 | 0.5 | 0.5 | 0.5 | 0.5 | 0.5 | 0.4 | 0.4 | 0.5 | 0.5 | 0.5 | 0.5 | 0.4 | 0.2 | 0.1 | | 0.5 | 0.5 | 0.5 | 0.4 | 0.5 | 0.5 | 0.3 | 0.3 | 0.5 | |
| R B B B | 0.4 | 0.4 | 0.4 | 0.4 | | 0.4 | 0.5 | 0.5 | 0.4 | 0.5 | 0.5 | 0.5 | 0.4 | 0.5 | 0.4 | 0.5 | 0.3 | 0.5 | | 0.1 | 0.4 | 0.4 | 0.3 | 0.5 | 0.4 | 0.4 | 0.4 | 0.5 |
| S A | 0.5 | 0.3 | 0.3 | 0.5 | 0.4 | 0.5 | 0.5 | 0.5 | 0.3 | 0.4 | 0.5 | 0.4 | 0.5 | 0.4 | 0.5 | 0.3 | 0.2 | 0.5 | 0.4 | | 0.1 | 0.5 | 0.5 | 0.4 | 0.5 | 0.3 | 0.3 | 0.4 |
| S B | 0.5 | 0.3 | 0.3 | 0.5 | 0.4 | 0.5 | 0.5 | 0.5 | 0.3 | 0.4 | 0.5 | 0.4 | 0.5 | 0.4 | 0.5 | 0.3 | 0.2 | 0.5 | 0.4 | 0.5 | | 0.1 | 0.5 | 0.4 | 0.5 | 0.3 | 0.3 | 0.4 |
| N S R | 0.5 | 0.2 | 0.2 | 0.5 | 0.3 | 0.5 | 0.4 | 0.4 | 0.3 | 0.3 | 0.4 | 0.4 | 0.4 | 0.4 | 0.5 | 0.3 | 0.1 | 0.4 | 0.3 | 0.5 | 0.5 | | 0.1 | 0.4 | 0.4 | 0.2 | 0.2 | 0.4 |
| S T a c h | 0.4 | 0.4 | 0.4 | 0.4 | 0.5 | 0.4 | 0.5 | 0.5 | 0.4 | 0.5 | 0.5 | 0.5 | 0.5 | 0.5 | 0.4 | 0.4 | 0.3 | 0.5 | 0.5 | 0.4 | 0.4 | 0.4 | | 0.1 | 0.5 | 0.4 | 0.4 | 0.5 |

| SVPB | 0.5 | 0.3 | 0.3 | 0.5 | 0.4 | 0.5 | 0.5 | 0.5 | 0.4 | 0.4 | 0.5 | 0.5 | 0.1 | | 0.5 | 0.5 | 0.4 | 0.2 | 0.5 | 0.4 | 0.5 | 0.5 | 0.4 | 0.5 | 0.1 | | 0.3 | 0.3 | 0.5 |
|---|---|---|---|---|---|---|---|---|---|---|---|---|---|---|---|---|---|---|---|---|---|---|---|---|---|---|---|---|---|
| Tab | 0.3 | 0.5 | 0.5 | 0.3 | 0.4 | 0.3 | 0.3 | 0.3 | 0.5 | 0.4 | 0.3 | 0.4 | 0.3 | 0.4 | 0.3 | 0.5 | 0.4 | 0.3 | 0.4 | 0.3 | 0.3 | 0.2 | 0.4 | 0.3 | 0.1 | | 0.5 | 0.4 | |
| TInv | 0.3 | 0.5 | 0.5 | 0.3 | 0.4 | 0.3 | 0.3 | 0.3 | 0.5 | 0.4 | 0.3 | 0.4 | 0.3 | 0.4 | 0.3 | 0.5 | 0.4 | 0.3 | 0.4 | 0.3 | 0.3 | 0.2 | 0.4 | 0.3 | 0.5 | 0.1 | | 0.4 | |
| VPB | 0.4 | 0.4 | 0.4 | 0.4 | 0.5 | 0.4 | 0.5 | 0.5 | 0.4 | 0.5 | 0.5 | 0.5 | 0.5 | 0.1 | | 0.4 | 0.4 | 0.3 | 0.5 | 0.5 | 0.4 | 0.4 | 0.4 | 0.5 | 0.5 | 0.4 | 0.4 | 0.1 | |

Suppose a given ground truth label is [AF, CRBBB, RBBB, RAD] and diagnosis predicted from a computational diagnostic model is [AF, AFL, NSR, RAD] for a diagnostic sample. First CRBBB and RBBB being equivalent is collapsed. Then, the first penalty is calculated according to equation 23, wherein $c_i$ is a diagnostic condition in predicted diagnosis. It is obtained from the step 208 by considering $n_i$=1 since there is only 1 occurrence of the predicted diagnosis, $y_i$=[AF, CRBBB, RBBB, RAD], and $\hat{x}_i$=[AF, AFL, NSR, RAD].

$$a_{ik} = \begin{cases} s_i & if\, c_i \in AF, RAD \\ -s_i & if\, c_i \in CRBB \\ s_i\left[\frac{1}{|y_k|}\left(\sum_{c_j \in y_k} w_{i,j}\right) - 1\right] & if\, c_i \in AFL, NSR \\ 0 & otherwise \end{cases} \quad (23)$$

Hence, the first penalty is $a_1$=[0, 1, ((0.5 + 0.4 + 0.3)/3 - 1), 0, -1, 0, 0, 0, 0, 0, 0, 0, 0, 0, 0, 0, 0, 0.25, 0, 0, ((0.3+0.2 + 0.4)/3-1), 0, 0, 0, 0, 0, 0 ] which is [0, 1, -0.6, 0, -1, 0, 0, 0, 0, 0, 0, 0, 0, 0, 0, 0, 0, 0.25, 0, 0, -0.7, 0, 0, 0, 0, 0, 0 ]. The second penalty calculated from the contradiction matrix is $b_1$ = [0, -1, -1, 0, -1, 0, 0, 0, 0, 0, 0, 0, 0, 0, 0, 0, 0, -0.25, 0, 0, -(0.25+0.25+0.25 + 0.25), 0, 0, 0, 0, 0, 0 ]. Thus, the pre-score corresponding to the diagnostic sample is t = -1.05 - 4.25 = -4.3. The total score of the multi-label multi-class computational diagnostic model is same as pre-score because there is only one diagnostic sample. The score of the perfect and null multi-label multi-class computational diagnostic models are 2.25 and -2.25 respectively. Hence, the metric can be computed as $\frac{-4.3 - (-2.25)}{2.25 - (-2.25)} = \frac{-2.05}{4.5} = -0.4555$.

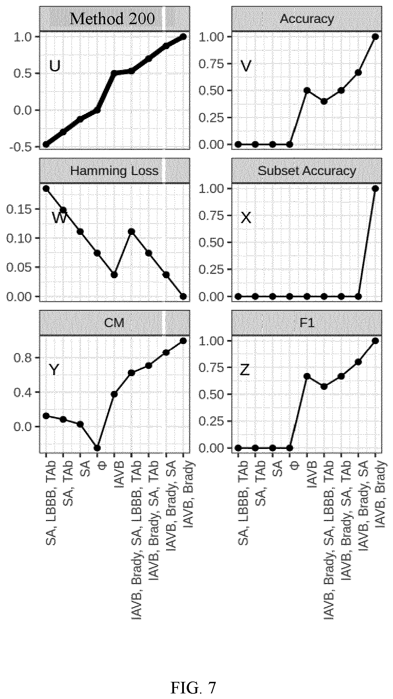

Table 5 provides a comparison of values calculated by method 200 and challenge metric for a number of predictions. FIG. 6 illustrates comparison of values calculated by method 200 with a plurality of metrics including accuracy, subset accuracy, hamming loss, F1 score and challenge metric for the ground truth and predicted diagnosis provided in Table 5. Similarly, FIG. 7 illustrates comparison of metrics for a different scenario the ground truth has two diagnostic conditions ({IAVB, Brady}). It can be observed that method 200 penalizes wrong diagnosis highest followed by missed and over diagnosis thereby satisfying the characteristics that are necessary for a good clinical metric.

Table 5

| Ground Truth | Prediction | Class of diagnosis | Metric of present disclosure | Challenge metric |
|---|---|---|---|---|
| CRBBB, AF, Qab | LAD, STach, Tinv | Wrong diagnosis | -0.23 | 0.253 |
| CRBBB, AF, Qab | LAD, Stach | Wrong diagnosis | -0.159 | 0.16 |
| CRBBB, AF, Qab | LAD | Wrong diagnosis | -0.081 | 0.048 |
| CRBBB, AF, Qab | φ | Missed diagnosis | 0 | -0.121 |
| CRBBB, AF, Qab | CRBBB | Missed diagnosis | 0.25 | 0.245 |
| CRBBB, AF, Qab | CRBBB, AF | Missed diagnosis | 0.75 | 0.633 |

| CRBBB, AF, Qab | CRBBB, AF, QAb, LAD, NSIVCB | Over diagnosis | 0.756 | 0.823 |
|---|---|---|---|---|
| CRBBB, AF, Qab | CRBBB, AF, QAb, LAD | Over diagnosis | 0.918 | 0.889 |
| CRBBB, AF, Qab | CRBBB, AF, Qab | Right diagnosis | 1 | 1 |

Table 6 illustrates examples where contradictions of diagnostic conditions are taken into consideration while evaluating the model. In these examples, NSR, AF and AF, SB are pairwise contradictory whereas AF, AFL are not. Also, (PR, CRBBB), (PR, Stach), (PR, SB), (PR, AF), (SB, Stach), (SB, AF) and (AF, Stach) are pairwise contradictory.

Table 6

| Ground Truth | Prediction | Metric Score |
|---|---|---|
| AF | AF | 1 |
| AF | NSR | -0.037 |
| AF | AF, NSR | 0.462 |
| AF | AF, AFL | 0.874 |
| AF | AF, NSR, SB | -0.072 |
| AF | AF, AFL, NSR | 0.337 |
| PR | φ | 0 |
| PR | CRBBB | -0.262 |
| PR | PR, CRBBB | 0.237 |
| PR | PR, CRBBB, Stach | -0.512 |
| PR | PR, CRBBB, STach, SB | -1.069 |
| PR | PR, CRBBB, STach, SB, AF | -2.195 |

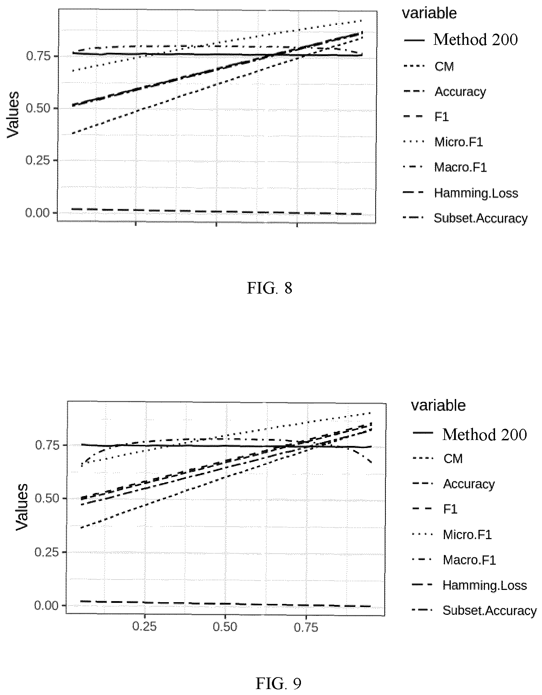

To test prevalence independence, a dataset with only two diagnostic conditions and a hypothetical classifier is considered. The classifier can detect condition A ({SA}) with 90% sensitivity (good performance), and condition B ({SB}) with 50% sensitivity (bad performance), and a fixed specificity of 100%. Then, the relative proportions of A, B is varied to study the performance of each measure. Ideally poor performance of the classifier in one class obfuscated by relative rarity of occurrence is not desired. However, from FIG. 8 it can be seen that only two metrics capture this fact. This experiment is repeated with 95% specificity and results are illustrated in FIG. 9. It can be seen that only metric of present disclosure is agnostic to relative prevalence of diagnostic conditions.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200) comprising:

   receiving (202), via one or more hardware processors, a dataset comprising a plurality of diagnostic samples and corresponding ground truth;
   predicting (204), via the one or more hardware processors, a diagnosis corresponding to each of the plurality of diagnostic samples using a multi-label multi-class computational diagnostic model, wherein the predicted diagnosis comprises one or more diagnostic conditions;
   classifying (206), via the one or more hardware processors, the predicted diagnosis in a class among a plurality of classes comprising: (i) a wrong diagnosis, (ii) a missed diagnosis, (iii) an over diagnosis and (iv) a right diagnosis;
   calculating (208), via the one or more hardware processors, a first penalty for the diagnosis corresponding to each of the plurality of diagnostic samples based on the class of the predicted diagnosis;
   calculating (210), via the one or more hardware processors, a second penalty for the diagnosis corresponding to each of the plurality of diagnostic samples based on a contradiction matrix which provides contradictory and non-contradictory pairs of diagnostic conditions, wherein the contradiction matrix is a square matrix of the diagnostic conditions and an entry $C_{ij}$ in a cell of the contradiction matrix is 1 if the diagnostic conditions $c_i$ and $c_j$ corresponding to its row and column do not occur together, and wherein the contradiction matrix is responsible for penalizing mutually exclusive diagnostic conditions;
   computing (212), via the one or more hardware processors, a pre-score for each of the plurality of diagnostic samples based on the corresponding first penalty and second penalty;
   obtaining (214), via the one or more hardware processors, a score corresponding to the multi-label multi-class computational diagnostic model by summing up the pre-score of each of the plurality of diagnostic samples; and
   evaluating (216), via the one or more hardware processors, the multi-label multi-class computational diagnostic model with a metric that is based on (i) the score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only.

2. The method of claim 1, wherein the predicted diagnosis for a diagnostic sample from among the plurality of diagnostic samples is classified as (i) the wrong diagnosis if the predicted diagnosis and ground truth corresponding to the diagnostic sample are disjoint, (ii) the missed diagnosis if the predicted diagnosis is a proper subset of the ground truth corresponding to the diagnostic sample, (iii) the over diagnosis if the ground truth corresponding to the diagnostic sample is a proper subset of the predicted diagnosis, or (iv) the right diagnosis otherwise.

3. The method of claim 1, wherein the first penalty is calculated by one of: (i) $\dfrac{s_i}{n_i}$ if the predicted diagnosis is a right diagnosis, (ii) $\dfrac{-s_i}{n_i}$ if the predicted diagnosis is a missed diagnosis, (iii) $\dfrac{s_i}{n^*}\left[\dfrac{1}{|y_k|}\left(\sum_{c_j \in y_k} w_{i,j}\right) - 1\right]$ if the predicted diagnosis is an over diagnosis, and (iv) 0 if the predicted diagnosis is a wrong diagnosis, wherein $s_i$ is pre-defined significance weight corresponding to class of diagnostic conditions in the dataset, $n_i$ is number of occurrences of the predicted diagnosis in the dataset, $n^* = max\{n_i | \forall c_i \in y_k\}$, $y_k$ is ground truth corresponding to the diagnostic sample for which prediction is done, $w_{i,j}$ is a weight matrix comprising cost of misclassification, and wherein assigning highest first penalty to wrong diagnosis followed by missed diagnosis and over diagnosis thereby imbibing risk aversion principle of clinical diagnosis.

4. The method of claim 1, wherein the second penalty is calculated as (i) $\dfrac{-1}{n_i} \sum_{\forall j \; s.t. \; c_j \in \hat{x}_k} s_j \cdot C_{ij}$ if $c_i$ is a diagnostic condition in the predicted diagnosis or (ii) 0 otherwise, wherein $n_i$ is number of occurrences of the predicted diagnosis in the dataset, $\hat{x}_k$ is the predicted diagnosis, $s_j$ is pre-defined significance weight corresponding to the class of diagnosis and $C_{ij}$ is an entry in the contradiction matrix.

5. A system (100), comprising:

   a memory (102) storing instructions;
   one or more Input/Output (I/O) interfaces (106); and
   one or more hardware processors (104) coupled to the memory (102) via the one or more communication interfaces (106), wherein the one or more hardware processors (104) are configured by the instructions to:

   receive a dataset comprising a plurality of diagnostic samples and corresponding ground truth;
   predict a diagnosis corresponding to each of the plurality of diagnostic samples using a multi-label multi-class computational diagnostic model, wherein the predicted diagnosis comprises one or more diagnostic conditions;
   classify the predicted diagnosis in a class among a plurality of classes comprising: (i) a wrong diagnosis, (ii) a missed diagnosis, (iii) an over diagnosis and (iv) a right diagnosis;
   calculate a first penalty for the diagnosis corresponding to each of the plurality of diagnostic samples based on the class of the predicted diagnosis;
   calculate a second penalty for the diagnosis corresponding to each of the plurality of diagnostic samples based on a contradiction matrix which provides contradictory and non-contradictory pairs of diagnostic conditions, wherein the contradiction matrix is a square matrix of the diagnostic conditions and an entry $C_{ij}$ in a cell of the contradiction matrix is 1 if the diagnostic conditions $c_i$ and $c_j$ corresponding to its row and column do not occur together, and wherein the contradiction matrix is responsible for penalizing mutually exclusive diagnostic conditions;
   compute a pre-score for each of the plurality of diagnostic samples based on the corresponding first penalty and second penalty;
   obtain a score corresponding to the multi-label multi-class computational diagnostic model by summing up the pre-score of each of the plurality of diagnostic samples; and
   evaluate the multi-label multi-class computational diagnostic model with a metric that is based on (i) the score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only.

6. The system of claim 5, wherein the predicted diagnosis for a diagnostic sample from among the plurality of diagnostic samples is classified as (i) the wrong diagnosis if the predicted diagnosis and ground truth corresponding to the diagnostic sample are disjoint, (ii) the missed diagnosis if the predicted diagnosis is a proper subset of the ground truth corresponding to the diagnostic sample, (iii) the over diagnosis if the ground truth corresponding to the diagnostic sample is a proper subset of the predicted diagnosis, or (iv) the right diagnosis otherwise.

7. The system of claim 5, wherein the first penalty is calculated by one of: (i) $\dfrac{s_i}{n_i}$ if the predicted diagnosis is a right diagnosis, (ii) $\dfrac{-s_i}{n_i}$ if the predicted diagnosis is a missed diagnosis, (iii) $\dfrac{s_i}{n^*}\left[\dfrac{1}{|y_k|}\left(\sum_{c_j \in y_k} w_{i,j}\right) - 1\right]$ if the predicted diagnosis is an over diagnosis, and (iv) 0 if the predicted diagnosis is a wrong diagnosis, wherein $s_i$ is pre-defined significance weight corresponding to class of diagnostic conditions in the dataset, $n_i$ is number of occurrences of the predicted diagnosis in the dataset, $n^* = max\{n_i | \forall c_i \in y_k\}$, $y_k$ is ground truth corresponding to the diagnostic sample for which prediction is done, $w_{i,j}$ is a weight matrix comprising cost of misclassification, and wherein assigning highest first penalty to wrong diagnosis followed by missed diagnosis and over diagnosis thereby imbibing risk aversion principle of clinical diagnosis.

8. The system of claim 5, wherein the second penalty is calculated as (i) $\frac{-1}{n_i} \sum_{\forall j \ s.t. \ c_j \in \hat{x}_k} s_j \cdot C_{ij}$ if $c_i$ is a diagnostic condition in the predicted diagnosis or (ii) 0 otherwise, wherein $n_i$ is number of occurrences of the predicted diagnosis in the dataset, $\hat{x}_k$ is the predicted diagnosis, $s_j$ is pre-defined significance weight corresponding to the class of diagnosis and $C_{ij}$ is an entry in the contradiction matrix.

9. One or more non-transitory machine-readable information storage mediums comprising one or more instructions which when executed by one or more hardware processors cause:

   receiving a dataset comprising a plurality of diagnostic samples and corresponding ground truth;
   predicting a diagnosis corresponding to each of the plurality of diagnostic samples using a multi-label multi-class computational diagnostic model, wherein the predicted diagnosis comprises one or more diagnostic conditions;
   classifying the predicted diagnosis in a class among a plurality of classes comprising: (i) a wrong diagnosis, (ii) a missed diagnosis, (iii) an over diagnosis and (iv) a right diagnosis;
   calculating a first penalty for the diagnosis corresponding to each of the plurality of diagnostic samples based on the class of the predicted diagnosis;
   calculating a second penalty for the diagnosis corresponding to each of the plurality of diagnostic samples based on a contradiction matrix which provides contradictory and non-contradictory pairs of diagnostic conditions, wherein the contradiction matrix is a square matrix of the diagnostic conditions and an entry $C_{ij}$ in a cell of the contradiction matrix is 1 if the diagnostic conditions $c_i$ and $c_j$ corresponding to its row and column do not occur together, and wherein the contradiction matrix is responsible for penalizing mutually exclusive diagnostic conditions;
   computing a pre-score for each of the plurality of diagnostic samples based on the corresponding first penalty and second penalty;
   obtaining a score corresponding to the multi-label multi-class computational diagnostic model by summing up the pre-score of each of the plurality of diagnostic samples; and
   evaluating the multi-label multi-class computational diagnostic model with a metric that is based on (i) the score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only.

10. The one or more non-transitory machine-readable information storage mediums of claim 9, wherein the predicted diagnosis for a diagnostic sample from among the plurality of diagnostic samples is classified as (i) the wrong diagnosis if the predicted diagnosis and ground truth corresponding to the diagnostic sample are disjoint, (ii) the missed diagnosis if the predicted diagnosis is a proper subset of the ground truth corresponding to the diagnostic sample, (iii) the over diagnosis if the ground truth corresponding to the diagnostic sample is a proper subset of the predicted diagnosis, or (iv) the right diagnosis otherwise.

11. The one or more non-transitory machine-readable information storage mediums of claim 9, wherein the first penalty is calculated by one of: (i) $\frac{s_i}{n_i}$ if the predicted diagnosis is a right diagnosis, (ii) $\frac{-s_i}{n_i}$ if the predicted diagnosis is a missed diagnosis, (iii) $\frac{s_i}{n^*}\left[\frac{1}{|y_k|}\left(\sum_{c_j \in y_k} w_{i,j}\right) - 1\right]$ if the predicted diagnosis is an over diagnosis, and (iv) 0 if the predicted diagnosis is a wrong diagnosis, wherein $s_i$ is pre-defined significance weight corresponding to class of diagnostic conditions in the dataset, $n_i$ is number of occurrences of the predicted diagnosis in the dataset, $n^* = max\{n_i | \forall c_i \in y_k\}$, $y_k$ is ground truth corresponding to the diagnostic sample for which prediction is done, $w_{i,j}$ is a weight matrix comprising cost of misclassification, and wherein assigning highest first penalty to wrong diagnosis followed by missed diagnosis and over diagnosis thereby imbibing risk aversion principle of clinical diagnosis.

12. The one or more non-transitory machine-readable information storage mediums of claim 9, wherein the second penalty is calculated as (i) $\frac{-1}{n_i} \sum_{\forall j \ s.t. \ c_j \in \hat{x}_k} s_j \cdot C_{ij}$ if $c_i$ is a diagnostic condition in the predicted diagnosis or (ii) 0

otherwise, wherein $n_i$ is number of occurrences of the predicted diagnosis in the dataset, $\hat{x}_k$ is the predicted diagnosis, $s_j$ is pre-defined significance weight corresponding to the class of diagnosis and $C_{ij}$ is an entry in the contradiction matrix.

**Patentansprüche**

1. Prozessorimplementiertes Verfahren (200), umfassend:

Empfangen (202), über einen oder mehrere Hardwareprozessoren, eines Datensatzes, der eine Mehrzahl von Diagnoseproben und entsprechende Ground Truth umfasst;
Vorhersagen (204), über den einen oder die mehreren Hardwareprozessoren, einer Diagnose, die jeder der Mehrzahl von Diagnoseproben entspricht, unter Verwendung eines Multi-Label-Multi-Klassen-Berechnungsdiagnosemodells, wobei die vorhergesagte Diagnose eine oder mehrere Diagnosebedingungen umfasst;
Klassifizieren (206), über den einen oder die mehreren Hardwareprozessoren, der vorhergesagten Diagnose in eine Klasse unter einer Mehrzahl von Klassen, umfassend: (i) eine falsche Diagnose, (ii) eine verpasste Diagnose, (iii) eine Überdiagnose und (iv) eine richtige Diagnose;
Berechnen (208), über den einen oder die mehreren Hardwareprozessoren, einer ersten Strafe für die Diagnose, die jeder der Mehrzahl von Diagnoseproben entspricht, basierend auf der Klasse der vorhergesagten Diagnose;
Berechnen (210), über den einen oder die mehreren Hardwareprozessoren, einer zweiten Strafe für die Diagnose, die jeder der Mehrzahl von Diagnoseproben entspricht, basierend auf einer Widerspruchsmatrix, die widersprüchliche und nicht widersprüchliche Paare von Diagnosebedingungen bereitstellt, wobei die Widerspruchsmatrix eine quadratische Matrix der Diagnosebedingungen ist und ein Eintrag $C_{ij}$ in einer Zelle der Widerspruchsmatrix 1 ist, wenn die Diagnosebedingungen $c_i$ und $c_j$, die ihrer Zeile und Spalte entsprechen, nicht zusammen auftreten, und wobei die Widerspruchsmatrix für das Bestrafen von sich gegenseitig ausschließenden Diagnosebedingungen verantwortlich ist;
Berechnen (212), über den einen oder die mehreren Hardwareprozessoren, einer Vorbewertung für jede der Mehrzahl von Diagnoseproben basierend auf der entsprechenden ersten Strafe und zweiten Strafe;
Erhalten (214), über den einen oder die mehreren Hardwareprozessoren, einer Bewertung, die dem Multi-Label-Multi-Klassen-Berechnungsdiagnosemodell entspricht, durch Summieren der Vorbewertung jeder der Mehrzahl von Diagnoseproben; und
Bewerten (216), über den einen oder die mehreren Hardwareprozessoren, des Multi-Label-Multi-Klassen-Berechnungsdiagnosemodells mit einer Metrik, die auf (i) der Bewertung, die dem Multi-Label-Multi-Klassen-Berechnungsdiagnosemodell entspricht, (ii) einer vorberechneten Bewertung eines perfekten Multi-Label-Multi-Klassen-Berechnungsdiagnosemodells, dessen Vorhersagen immer zu der richtigen Diagnoseklasse gehören, und (iii) einer vorberechneten Bewertung eines Null-Multi-Label-Multi-Klassen-Berechnungsdiagnosemodells, das nur Null oder 0 vorhersagt, basiert.

2. Verfahren nach Anspruch 1, wobei die vorhergesagte Diagnose für eine Diagnoseprobe aus der Mehrzahl von Diagnoseproben klassifiziert wird als (i) die falsche Diagnose, wenn die vorhergesagte Diagnose und die Grundwahrheit, die der Diagnoseprobe entspricht, disjunkt sind, (ii) die verpasste Diagnose, wenn die vorhergesagte Diagnose eine richtige Teilmenge der Grundwahrheit ist, die der Diagnoseprobe entspricht, (iii) die Überdiagnose, wenn die Grundwahrheit, die der Diagnoseprobe entspricht, eine richtige Teilmenge der vorhergesagten Diagnose ist, oder (iv) andernfalls die richtige Diagnose.

3. Verfahren nach Anspruch 1, wobei die erste Strafe berechnet wird durch eines von: (i) $\frac{s_i}{n_i}$ wenn die vorhergesagte Diagnose eine richtige Diagnose ist, (ii) $\frac{-s_i}{n_i}$ wenn die vorhergesagte Diagnose eine verpasste Diagnose ist, (iii) $\frac{s_i}{n^*}\left[\frac{1}{|y_k|}\left(\sum_{c_j \in y_k} w_{i,j}\right) - 1\right]$ wenn die vorhergesagte Diagnose eine Überdiagnose ist, und (iv) 0 wenn die vorhergesagte Diagnose eine falsche Diagnose ist, wobei $s_i$ ein vordefiniertes Signifikanzgewicht ist, das der Klasse von Diagnosebedingungen in dem Datensatz entspricht, $n_i$ die Anzahl von Vorkommen der vorhergesagten Diagnose in dem Datensatz ist, $n^* = max\{n_i | \forall c_i \in y_k\}$, $y_k$ die Grundwahrheit ist, die der Diagnoseprobe entspricht, für die die Vorhersage durchgeführt wird, $w_{i,j}$ eine Gewichtsmatrix ist, die Kosten einer Fehlklassifizierung umfasst, und wobei das Zuweisen der höchsten ersten Strafe zu einer falschen Diagnose gefolgt von einer verpassten Diagnose und einer Überdiagnose dadurch das Risikoabwendungsprinzip der klinischen Diagnose aufnimmt.

4. Verfahren nach Anspruch 1, wobei die zweite Strafe berechnet wird als (i) $\frac{-1}{n_i}\sum_{\forall j \, s.t. \, c_j \in \hat{x}_k} s_j \cdot C_{ij}$ wenn $c_i$ eine Diagnosebedingung in der vorhergesagten Diagnose ist oder (ii) andernfalls 0, wobei $n_i$ die Anzahl von Vorkommen der vorhergesagten Diagnose in dem Datensatz ist, $\hat{x}_k$ die vorhergesagte Diagnose ist, $s_j$ ein vordefiniertes Signifikanzgewicht ist, das der Diagnoseklasse entspricht, und $C_{ij}$ ein Eintrag in der Widerspruchsmatrix ist.

5. System (100), umfassend:

   einen Speicher (102), der Anweisungen speichert;
   eine oder mehrere Eingabe/Ausgabe(E/A)-Schnittstellen (106); und
   einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

      Empfangen eines Datensatzes, der eine Mehrzahl von Diagnoseproben und entsprechende Ground Truth umfasst;
      Vorhersagen einer Diagnose, die jeder der Mehrzahl von Diagnoseproben entspricht, unter Verwendung eines Multi-Label-Multi-Klassen-Berechnungsdiagnosemodells, wobei die vorhergesagte Diagnose eine oder mehrere Diagnosebedingungen umfasst;
      Klassifizieren der vorhergesagten Diagnose in eine Klasse unter einer Mehrzahl von Klassen, umfassend: (i) eine falsche Diagnose, (ii) eine verpasste Diagnose, (iii) eine Überdiagnose und (iv) eine richtige Diagnose;
      Berechnen einer ersten Strafe für die Diagnose, die jeder der Mehrzahl von Diagnoseproben entspricht, basierend auf der Klasse der vorhergesagten Diagnose;
      Berechnen einer zweiten Strafe für die Diagnose, die jeder der Mehrzahl von Diagnoseproben entspricht, basierend auf einer Widerspruchsmatrix, die widersprüchliche und nicht widersprüchliche Paare von Diagnosebedingungen bereitstellt, wobei die Widerspruchsmatrix eine quadratische Matrix der Diagnosebedingungen ist und ein Eintrag $C_{ij}$ in einer Zelle der Widerspruchsmatrix 1 ist, wenn die Diagnosebedingungen $c_i$ und $c_j$, die ihrer Zeile und Spalte entsprechen, nicht zusammen auftreten, und wobei die Widerspruchsmatrix für das Bestrafen von sich gegenseitig ausschließenden Diagnosebedingungen verantwortlich ist;
      Berechnen einer Vorbewertung für jede der Mehrzahl von Diagnoseproben basierend auf der entsprechenden ersten Strafe und zweiten Strafe;
      Erhalten einer Bewertung, die dem Multi-Label-Multi-Klassen-Berechnungsdiagnosemodell entspricht, durch Summieren der Vorbewertung jeder der Mehrzahl von Diagnoseproben; und
      Bewerten des Multi-Label-Multi-Klassen-Berechnungsdiagnosemodells mit einer Metrik, die auf (i) der Bewertung, die dem Multi-Label-Multi-Klassen-Berechnungsdiagnosemodell entspricht, (ii) einer vorberechneten Bewertung eines perfekten Multi-Label-Multi-Klassen-Berechnungsdiagnosemodells, dessen Vorhersagen immer zu der richtigen Diagnoseklasse gehören, und (iii) einer vorberechneten Bewertung eines Null-Multi-Label-Multi-Klassen-Berechnungsdiagnosemodells, das nur Null oder 0 vorhersagt, basiert.

6. System nach Anspruch 5, wobei die vorhergesagte Diagnose für eine Diagnoseprobe aus der Mehrzahl von Diagnoseproben klassifiziert wird als (i) die falsche Diagnose, wenn die vorhergesagte Diagnose und die Grundwahrheit, die der Diagnoseprobe entspricht, disjunkt sind, (ii) die verpasste Diagnose, wenn die vorhergesagte Diagnose eine richtige Teilmenge der Grundwahrheit ist, die der Diagnoseprobe entspricht, (iii) die Überdiagnose, wenn die Grundwahrheit, die der Diagnoseprobe entspricht, eine richtige Teilmenge der vorhergesagten Diagnose ist, oder (iv) andernfalls die richtige Diagnose.

7. System nach Anspruch 5, wobei die erste Strafe berechnet wird durch eines von: (i) $\frac{s_i}{n_i}$ wenn die vorhergesagte Diagnose eine richtige Diagnose ist, (ii) $\frac{-s_i}{n_i}$ wenn die vorhergesagte Diagnose eine verpasste Diagnose ist, (iii) $\frac{s_i}{n^*}\left[\frac{1}{|y_k|}\left(\sum_{c_j \in y_k} w_{i,j}\right) - 1\right]$ wenn die vorhergesagte Diagnose eine Überdiagnose ist, und (iv) 0 wenn die vorhergesagte Diagnose eine falsche Diagnose ist, wobei $s_i$ ein vordefiniertes Signifikanzgewicht ist, das der Klasse von Diagnosebedingungen in dem Datensatz entspricht, $n_i$ die Anzahl von Vorkommen der vorhergesagten Diagnose

22

in dem Datensatz ist, n* = $max\{n_i|\forall c_i \in y_k\}$, $y_k$ die Grundwahrheit ist, die der Diagnoseprobe entspricht, für die die Vorhersage durchgeführt wird, $w_{i,j}$ eine Gewichtsmatrix ist, die Kosten einer Fehlklassifizierung umfasst, und wobei das Zuweisen der höchsten ersten Strafe zu einer falschen Diagnose gefolgt von einer verpassten Diagnose und einer Überdiagnose dadurch das Risikoabwendungsprinzip der klinischen Diagnose aufnimmt.

8. System nach Anspruch 5, wobei die zweite Strafe berechnet wird als (i) $\frac{-1}{n_i}\sum_{\forall j\ s.t.\ c_j\in\hat{x}_k} s_j \cdot C_{ij}$ wenn $c_i$ eine

Diagnosebedingung in der vorhergesagten Diagnose ist oder (ii) andernfalls 0, wobei $n_i$ die Anzahl von Vorkommen der vorhergesagten Diagnose in dem Datensatz ist, $\hat{x}_k$ die vorhergesagte Diagnose ist, $s_j$ ein vordefiniertes Signifikanzgewicht ist, das der Diagnoseklasse entspricht, und $C_{ij}$ ein Eintrag in der Widerspruchsmatrix ist.

9. Ein oder mehrere nichtflüchtige maschinenlesbare Informationsspeichermedien, die eine oder mehrere Anweisungen umfassen, die, wenn sie durch einen oder mehrere Hardwareprozessoren ausgeführt werden, bewirken:

   Empfangen eines Datensatzes, der eine Mehrzahl von Diagnoseproben und entsprechende Ground Truth umfasst;
   Vorhersagen einer Diagnose, die jeder der Mehrzahl von Diagnoseproben entspricht, unter Verwendung eines Multi-Label-Multi-Klassen-Berechnungsdiagnosemodells, wobei die vorhergesagte Diagnose eine oder mehrere Diagnosebedingungen umfasst;
   Klassifizieren der vorhergesagten Diagnose in eine Klasse unter einer Mehrzahl von Klassen, umfassend: (i) eine falsche Diagnose, (ii) eine verpasste Diagnose, (iii) eine Überdiagnose und (iv) eine richtige Diagnose;
   Berechnen einer ersten Strafe für die Diagnose, die jeder der Mehrzahl von Diagnoseproben entspricht, basierend auf der Klasse der vorhergesagten Diagnose;
   Berechnen einer zweiten Strafe für die Diagnose, die jeder der Mehrzahl von Diagnoseproben entspricht, basierend auf einer Widerspruchsmatrix, die widersprüchliche und nicht widersprüchliche Paare von Diagnosebedingungen bereitstellt, wobei die Widerspruchsmatrix eine quadratische Matrix der Diagnosebedingungen ist und ein Eintrag $C_{ij}$ in einer Zelle der Widerspruchsmatrix 1 ist, wenn die Diagnosebedingungen $c_i$ und $c_j$, die ihrer Zeile und Spalte entsprechen, nicht zusammen auftreten, und wobei die Widerspruchsmatrix für das Bestrafen von sich gegenseitig ausschließenden Diagnosebedingungen verantwortlich ist;
   Berechnen einer Vorbewertung für jede der Mehrzahl von Diagnoseproben basierend auf der entsprechenden ersten Strafe und zweiten Strafe;
   Erhalten einer Bewertung, die dem Multi-Label-Multi-Klassen-Berechnungsdiagnosemodell entspricht, durch Summieren der Vorbewertung jeder der Mehrzahl von Diagnoseproben; und
   Bewerten des Multi-Label-Multi-Klassen-Berechnungsdiagnosemodells mit einer Metrik, die auf (i) der Bewertung, die dem Multi-Label-Multi-Klassen-Berechnungsdiagnosemodell entspricht, (ii) einer vorberechneten Bewertung eines perfekten Multi-Label-Multi-Klassen-Berechnungsdiagnosemodells, dessen Vorhersagen immer zu der richtigen Diagnoseklasse gehören, und (iii) einer vorberechneten Bewertung eines Null-Multi-Label-Multi-Klassen-Berechnungsdiagnosemodells, das nur Null oder 0 vorhersagt, basiert.

10. Ein oder mehrere nichtflüchtige maschinenlesbare Informationsspeichermedien nach Anspruch 9, wobei die vorhergesagte Diagnose für eine Diagnoseprobe aus der Mehrzahl von Diagnoseproben klassifiziert wird als (i) die falsche Diagnose, wenn die vorhergesagte Diagnose und die Grundwahrheit, die der Diagnoseprobe entspricht, disjunkt sind, (ii) die verpasste Diagnose, wenn die vorhergesagte Diagnose eine richtige Teilmenge der Grundwahrheit ist, die der Diagnoseprobe entspricht, (iii) die Überdiagnose, wenn die Grundwahrheit, die der Diagnoseprobe entspricht, eine richtige Teilmenge der vorhergesagten Diagnose ist, oder (iv) andernfalls die richtige Diagnose.

11. Ein oder mehrere nichtflüchtige maschinenlesbare Informationsspeichermedien nach Anspruch 9, wobei die erste Strafe berechnet wird durch eines von: (i) $\frac{s_i}{n_i}$ wenn die vorhergesagte Diagnose eine richtige Diagnose ist, (ii) $\frac{-s_i}{n_i}$

wenn die vorhergesagte Diagnose eine verpasste Diagnose ist, (iii) $\frac{s_i}{n^*}\left[\frac{1}{|y_k|}\left(\sum_{c_j\in y_k} w_{i,j}\right)-1\right]$ wenn die vorhergesagte Diagnose eine Überdiagnose ist, und (iv) 0 wenn die vorhergesagte Diagnose eine falsche Diagnose ist, wobei $s_i$ ein vordefiniertes Signifikanzgewicht ist, das der Klasse von Diagnosebedingungen in dem Datensatz entspricht, $n_i$ die Anzahl von Vorkommen der vorhergesagten Diagnose in dem Datensatz ist, n* = $max\{n_i|\forall c_i \in y_k\}$, $y_k$ die Grundwahrheit ist, die der Diagnoseprobe entspricht, für die die Vorhersage durchgeführt wird, $w_{i,j}$ eine Gewichtsmatrix ist, die Kosten einer Fehlklassifizierung umfasst, und wobei das Zuweisen der höchsten ersten Strafe zu einer falschen Diagnose gefolgt von einer verpassten Diagnose und einer Überdiagnose dadurch das Risikoabwendungsprinzip der klinischen Diagnose aufnimmt.

12. Ein oder mehrere nichtflüchtige maschinenlesbare Informationsspeichermedien nach Anspruch 9, wobei die zweite Strafe berechnet wird als (i) $\frac{-1}{n_i} \sum_{\forall j \, s.t. \, c_j \in \hat{x}_k} s_j \cdot C_{ij}$ eine Diagnosebedingung in der vorhergesagten Diagnose ist oder (ii) andernfalls 0, wobei $n_i$ die Anzahl von Vorkommen der vorhergesagten Diagnose in dem Datensatz ist, $\hat{x}_k$ die vorhergesagte Diagnose ist, $s_j$ ein vordefiniertes Signifikanzgewicht ist, das der Diagnoseklasse entspricht, und $C_{ij}$ ein Eintrag in der Widerspruchsmatrix ist.

**Revendications**

1. Procédé mis en œuvre par processeur (200) comprenant :

   la réception (202), via un ou plusieurs processeurs matériels, d'un ensemble de données comprenant une pluralité d'échantillons de diagnostic et une vérité de terrain correspondante ;
   la prédiction (204), via les un ou plusieurs processeurs matériels, d'un diagnostic correspondant à chacun de la pluralité d'échantillons de diagnostic en utilisant un modèle de diagnostic informatique multi-étiquettes multi-classes, dans lequel le diagnostic prédit comprend une ou plusieurs conditions de diagnostic ;
   la classification (206), via les un ou plusieurs processeurs matériels, du diagnostic prédit dans une classe parmi une pluralité de classes comprenant : (i) un mauvais diagnostic, (ii) un diagnostic manqué, (iii) un surdiagnostic et (iv) un diagnostic correct ;
   le calcul (208), via les un ou plusieurs processeurs matériels, d'une première pénalité pour le diagnostic correspondant à chacun de la pluralité d'échantillons de diagnostic sur la base de la classe du diagnostic prédit ;
   le calcul (210), via les un ou plusieurs processeurs matériels, d'une seconde pénalité pour le diagnostic correspondant à chacun de la pluralité d'échantillons de diagnostic sur la base d'une matrice de contradiction qui fournit des paires contradictoires et non contradictoires de conditions de diagnostic, dans lequel la matrice de contradiction est une matrice carrée des conditions de diagnostic et une entrée $C_{ij}$ dans une cellule de la matrice de contradiction est 1 si les conditions de diagnostic $c_i$ et $c_j$ correspondant à sa ligne et sa colonne ne se produisent pas ensemble, et dans lequel la matrice de contradiction est responsable de la pénalisation de conditions de diagnostic mutuellement exclusives ;
   le calcul (212), via les un ou plusieurs processeurs matériels, d'un pré-score pour chacun de la pluralité d'échantillons de diagnostic sur la base de la première pénalité et de la seconde pénalité correspondantes ;
   l'obtention (214), via les un ou plusieurs processeurs matériels, d'un score correspondant au modèle de diagnostic informatique multi-étiquettes multi-classes en additionnant le pré-score de chacun de la pluralité d'échantillons de diagnostic ; et
   l'évaluation (216), via les un ou plusieurs processeurs matériels, du modèle de diagnostic informatique multi-étiquettes multi-classes avec une métrique qui est basée sur (i) le score correspondant au modèle de diagnostic informatique multi-étiquettes multi-classes, (ii) un score précalculé d'un modèle de diagnostic informatique multi-étiquettes multi-classes parfait dont les prédictions appartiennent toujours à la classe de diagnostic correct et (iii) un score précalculé d'un modèle de diagnostic informatique multi-étiquettes multi-classes nul qui prédit null ou 0 uniquement.

2. Procédé selon la revendication 1, dans lequel le diagnostic prédit pour un échantillon de diagnostic parmi la pluralité d'échantillons de diagnostic est classé comme (i) le mauvais diagnostic si le diagnostic prédit et la vérité de terrain correspondant à l'échantillon de diagnostic sont disjoints, (ii) le diagnostic manqué si le diagnostic prédit est un sous-ensemble approprié de la vérité de terrain correspondant à l'échantillon de diagnostic, (iii) le surdiagnostic si la vérité de terrain correspondant à l'échantillon de diagnostic est un sous-ensemble approprié du diagnostic prédit, ou (iv) le diagnostic correct sinon.

3. Procédé selon la revendication 1, dans lequel la première pénalité est calculée par l'un parmi : (i) $\frac{s_i}{n_i}$ si le diagnostic prédit est un diagnostic correct, (ii) $\frac{-s_i}{n_i}$ si le diagnostic prédit est un diagnostic manqué, (iii) $\frac{s_i}{n^*}\left[\frac{1}{|y_k|}\left(\sum_{c_j \in y_k} w_{i,j}\right) - 1\right]$ si le diagnostic prédit est un surdiagnostic, et (iv) 0 si le diagnostic prédit est un mauvais diagnostic, dans lequel $s_i$ est un poids d'importance prédéfini correspondant à une classe de conditions de diagnostic dans l'ensemble de données, $n_i$ est un nombre d'occurrences du diagnostic prédit dans l'ensemble de données, $n^* = max\{n_i | \forall c_i \in y_k\}$, $y_k$ est une vérité de terrain correspondant à l'échantillon de diagnostic pour lequel la prédiction est faite, $w_{i,j}$ est une matrice de poids comprenant un coût de mauvaise classification, et dans lequel l'attribution de la première pénalité la plus élevée à un mauvais diagnostic suivi d'un diagnostic manqué et d'un surdiagnostic reflète ainsi le principe d'aversion de risque de diagnostic clinique.

4. Procédé selon la revendication 1, dans lequel la seconde pénalité est calculée comme (i) $\frac{-1}{n_i} \sum_{\forall j \ s.t. \ c_j \in \hat{x}_k} s_j \cdot C_{ij}$ si $c_i$ est une condition de diagnostic dans le diagnostic prédit ou (ii) 0 sinon, dans lequel $n_i$ est un nombre d'occurrences du diagnostic prédit dans l'ensemble de données, $\hat{x}_k$ est le diagnostic prédit, $s_j$ est un poids d'importance prédéfini correspondant à la classe de diagnostic et $C_{ij}$ est une entrée dans la matrice de contradiction.

5. Système (100), comprenant :

  une mémoire (102) stockant des instructions ;
  une ou plusieurs interfaces d'entrée/sortie (E/S) (106) ; et
  un ou plusieurs processeurs matériels (104) couplés à la mémoire (102) via les une ou plusieurs interfaces de communication (106), dans lequel les un ou plusieurs processeurs matériels (104) sont configurés par les instructions pour :

    recevoir un ensemble de données comprenant une pluralité d'échantillons de diagnostic et une vérité de terrain correspondante ;
    prédire un diagnostic correspondant à chacun de la pluralité d'échantillons de diagnostic en utilisant un modèle de diagnostic informatique multi-étiquettes multi-classes, dans lequel le diagnostic prédit comprend une ou plusieurs conditions de diagnostic ;
    classer le diagnostic prédit dans une classe parmi une pluralité de classes comprenant : (i) un mauvais diagnostic, (ii) un diagnostic manqué, (iii) un surdiagnostic et (iv) un diagnostic correct ;
    calculer une première pénalité pour le diagnostic correspondant à chacun de la pluralité d'échantillons de diagnostic sur la base de la classe du diagnostic prédit ;
    calculer une seconde pénalité pour le diagnostic correspondant à chacun de la pluralité d'échantillons de diagnostic sur la base d'une matrice de contradiction qui fournit des paires contradictoires et non contradictoires de conditions de diagnostic, dans lequel la matrice de contradiction est une matrice carrée des conditions de diagnostic et une entrée $C_{ij}$ dans une cellule de la matrice de contradiction est 1 si les conditions de diagnostic $c_i$ et $c_j$ correspondant à sa ligne et sa colonne ne se produisent pas ensemble, et dans lequel la matrice de contradiction est responsable de la pénalisation de conditions de diagnostic mutuellement exclusives ;
    calculer un pré-score pour chacun de la pluralité d'échantillons de diagnostic sur la base de la première pénalité et de la seconde pénalité correspondantes ;
    obtenir un score correspondant au modèle de diagnostic informatique multi-étiquettes multi-classes en additionnant le pré-score de chacun de la pluralité d'échantillons de diagnostic ; et
    évaluer le modèle de diagnostic informatique multi-étiquettes multi-classes avec une métrique qui est basée sur (i) le score correspondant au modèle de diagnostic informatique multi-étiquettes multi-classes, (ii) un score précalculé d'un modèle de diagnostic informatique multi-étiquettes multi-classes parfait dont les prédictions appartiennent toujours à la classe de diagnostic correct et (iii) un score précalculé d'un modèle de diagnostic informatique multi-étiquettes multi-classes nul qui prédit null ou 0 uniquement.

6. Système selon la revendication 5, dans lequel le diagnostic prédit pour un échantillon de diagnostic parmi la pluralité d'échantillons de diagnostic est classé comme (i) le mauvais diagnostic si le diagnostic prédit et la vérité de terrain correspondant à l'échantillon de diagnostic sont disjoints, (ii) le diagnostic manqué si le diagnostic prédit est un sous-ensemble approprié de la vérité de terrain correspondant à l'échantillon de diagnostic, (iii) le surdiagnostic si la vérité de terrain correspondant à l'échantillon de diagnostic est un sous-ensemble approprié du diagnostic prédit, ou (iv) le diagnostic correct sinon.

7. Système selon la revendication 5, dans lequel la première pénalité est calculée par l'un parmi : (i) $\frac{s_i}{n_i}$ si le diagnostic prédit est un diagnostic correct, (ii) $\frac{-s_i}{n_i}$ si le diagnostic prédit est un diagnostic manqué, (iii) $\frac{s_i}{n^*}\left[\frac{1}{|y_k|}\left(\sum_{c_j \in y_k} w_{i,j}\right) - 1\right]$ si le diagnostic prédit est un surdiagnostic, et (iv) 0 si le diagnostic prédit est un mauvais diagnostic, dans lequel $s_i$ est un poids d'importance prédéfini correspondant à une classe de conditions de diagnostic dans l'ensemble de données, $n_i$ est un nombre d'occurrences du diagnostic prédit dans l'ensemble de données, $n^* = max\{n_i | \forall c_i \in y_k\}$, $y_k$ est une vérité de terrain correspondant à l'échantillon de diagnostic pour lequel la prédiction est faite, $w_{i,j}$ est une matrice de poids comprenant un coût de mauvaise classification, et dans lequel l'attribution de la première pénalité la plus élevée à un mauvais diagnostic suivi d'un diagnostic manqué et d'un surdiagnostic reflète ainsi le principe d'aversion de risque de diagnostic clinique.

8. Système selon la revendication 5, dans lequel la seconde pénalité est calculée comme (i) $\frac{-1}{n_i}\sum_{\forall j \ s.t. \ c_j \in \hat{x}_k} s_j \cdot C_{ij}$

si $c_i$ est une condition de diagnostic dans le diagnostic prédit ou (ii) 0 sinon, dans lequel $n_i$ est un nombre d'occurrences du diagnostic prédit dans l'ensemble de données, $\hat{x}_k$ est le diagnostic prédit, $s_j$ est un poids d'importance prédéfini correspondant à la classe de diagnostic et $C_{ij}$ est une entrée dans la matrice de contradiction.

9. Un ou plusieurs supports de stockage d'informations lisibles par machine non transitoires comprenant une ou plusieurs instructions qui, lorsqu'elles sont exécutées par un ou plusieurs processeurs matériels, amènent :

la réception d'un ensemble de données comprenant une pluralité d'échantillons de diagnostic et une vérité de terrain correspondante ;
la prédiction d'un diagnostic correspondant à chacun de la pluralité d'échantillons de diagnostic en utilisant un modèle de diagnostic informatique multi-étiquettes multi-classes, dans lequel le diagnostic prédit comprend une ou plusieurs conditions de diagnostic ;
la classification du diagnostic prédit dans une classe parmi une pluralité de classes comprenant : (i) un mauvais diagnostic, (ii) un diagnostic manqué, (iii) un surdiagnostic et (iv) un diagnostic correct ;
le calcul d'une première pénalité pour le diagnostic correspondant à chacun de la pluralité d'échantillons de diagnostic sur la base de la classe du diagnostic prédit ;
le calcul d'une seconde pénalité pour le diagnostic correspondant à chacun de la pluralité d'échantillons de diagnostic sur la base d'une matrice de contradiction qui fournit des paires contradictoires et non contradictoires de conditions de diagnostic, dans lequel la matrice de contradiction est une matrice carrée des conditions de diagnostic et une entrée $C_{ij}$ dans une cellule de la matrice de contradiction est 1 si les conditions de diagnostic $c_i$ et $c_j$ correspondant à sa ligne et sa colonne ne se produisent pas ensemble, et dans lequel la matrice de contradiction est responsable de la pénalisation de conditions de diagnostic mutuellement exclusives ;
le calcul d'un pré-score pour chacun de la pluralité d'échantillons de diagnostic sur la base de la première pénalité et de la seconde pénalité correspondantes ;
l'obtention d'un score correspondant au modèle de diagnostic informatique multi-étiquettes multi-classes en additionnant le pré-score de chacun de la pluralité d'échantillons de diagnostic ; et
l'évaluation du modèle de diagnostic informatique multi-étiquettes multi-classes avec une métrique qui est basée sur (i) le score correspondant au modèle de diagnostic informatique multi-étiquettes multi-classes, (ii) un score précalculé d'un modèle de diagnostic informatique multi-étiquettes multi-classes parfait dont les prédictions appartiennent toujours à la classe de diagnostic correct et (iii) un score précalculé d'un modèle de diagnostic informatique multi-étiquettes multi-classes nul qui prédit null ou 0 uniquement.

10. Un ou plusieurs supports de stockage d'informations lisibles par machine non transitoires selon la revendication 9, dans lequel le diagnostic prédit pour un échantillon de diagnostic parmi la pluralité d'échantillons de diagnostic est classé comme (i) le mauvais diagnostic si le diagnostic prédit et la vérité de terrain correspondant à l'échantillon de diagnostic sont disjoints, (ii) le diagnostic manqué si le diagnostic prédit est un sous-ensemble approprié de la vérité de terrain correspondant à l'échantillon de diagnostic, (iii) le surdiagnostic si la vérité de terrain correspondant à l'échantillon de diagnostic est un sous-ensemble approprié du diagnostic prédit, ou (iv) le diagnostic correct sinon.

11. Un ou plusieurs supports de stockage d'informations lisibles par machine non transitoires selon la revendication 9, dans lequel la première pénalité est calculée par l'un parmi : (i) $\frac{s_i}{n_i}$ si le diagnostic prédit est un diagnostic correct, (ii) $\frac{-s_i}{n_i}$ si le diagnostic prédit est un diagnostic manqué, (iii) $\frac{s_i}{n^*}\left[\frac{1}{|y_k|}\left(\sum_{c_j \in y_k} w_{i,j}\right) - 1\right]$ si le diagnostic prédit est un surdiagnostic, et (iv) 0 si le diagnostic prédit est un mauvais diagnostic, dans lequel $s_i$ est un poids d'importance prédéfini correspondant à une classe de conditions de diagnostic dans l'ensemble de données, $n_i$ est un nombre d'occurrences du diagnostic prédit dans l'ensemble de données, $n^* = max\{n_i | \forall c_i \in y_k\}$, $y_k$ est une vérité de terrain correspondant à l'échantillon de diagnostic pour lequel la prédiction est faite, $w_{i,j}$ est une matrice de poids comprenant un coût de mauvaise classification, et dans lequel l'attribution de la première pénalité la plus élevée à un mauvais diagnostic suivi d'un diagnostic manqué et d'un surdiagnostic reflète ainsi le principe d'aversion de risque de diagnostic clinique.

12. Un ou plusieurs supports de stockage d'informations lisibles par machine non transitoires selon la revendication 9, dans lequel la seconde pénalité est calculée comme (i) $\frac{-1}{n_i} \sum_{\forall j\ s.t.\ c_j \in \hat{x}_k} s_j \cdot C_{ij}$ si $c_i$ est une condition de diagnostic dans le diagnostic prédit ou (ii) 0 sinon, dans lequel $n_i$ est un nombre d'occurrences du diagnostic prédit dans l'ensemble de données, $\hat{x}_k$ est le diagnostic prédit, $s_j$ est un poids d'importance prédéfini correspondant à la classe de diagnostic et $C_{ij}$ est une entrée dans la matrice de contradiction.

SYSTEM

100

MEMORY

102

DATABASE

108

HARDWARE

PROCESSOR(S)

104

I/O INTERFACE(S)

106

FIG. 1

200

Receiving a dataset comprising a plurality of diagnostic samples and corresponding ground truth → 202

Predicting a diagnosis corresponding to each of the plurality of diagnostic samples using a multi-label multi-class computational diagnostic model → 204

Classifying the predicted diagnosis in a class among a plurality of classes comprising: (i) a wrong diagnosis, (ii) a missed diagnosis, (iii) an over diagnosis and (iv) a right diagnosis → 206

Calculating a first penalty for the diagnosis corresponding to each of the plurality of diagnostic samples based on the class of the predicted diagnosis → 208

Calculating a second penalty for the diagnosis corresponding to each of the plurality of diagnostic samples based on a contradiction matrix → 210

A

FIG. 2A

200

(A)

Computing a pre-score for each of the plurality of diagnostic samples based on the corresponding first penalty and second penalty — 212

Obtaining a score corresponding to the multi-label multi-class computational diagnostic model by summing up the pre-scores of each of the plurality of diagnostic samples — 214

Evaluating the multi-label multi-class computational diagnostic model with a metric that is based on (i) the score corresponding to the multi-label multi-class computational diagnostic model, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only — 216

FIG. 2B

Input: Diagnostic samples and corresponding ground truth

Predict a diagnosis corresponding to each diagnostic sample using a multi-label multi-class computational diagnostic model

Classify the predicted diagnosis

Calculate first penalty

Calculate second penalty

Compute a pre-score for each diagnostic sample

Compute total score as sum of pre-scores of the diagnostic samples

Evaluate the multi-label multi-class computational diagnostic model with a metric that is based on (i) the total score, (ii) a pre-computed score of a perfect multi-label multi-class computational diagnostic model whose predictions always belong to the right diagnosis class and (iii) a pre-computed score of a null multi-label multi-class computational diagnostic model which predicts null or 0 only

FIG. 3

FIG. 4

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221052587

**Non-patent literature cited in the description**

- **CAI WENUJIE et al.** *Classification of multi-lead ECG with deep residual convolutional neural networks*
- **JIMENEZ-SERRANO SANTIAGO et al.** *From 12 to 1 ECG lead: multiple cardiac condition detection mixing a hybrid machine learning approach with a one-versus-rest classification strategy*
- **XU ZHUOYANG et al.** *Abnormality classification from electrocardiograms with various lead combinations*
- **REYNA MATTHEW A et al.** *Issues in the automated classification of multilead ecgs using heterogeneous labels and populations*
- **HICKS SA et al.** On evaluation metrics for medical applications of artificial intelligence. *Scientific Reports*, 2022, 5979
- **ALDAY EAP et al.** Classification of 12-lead ECGs: the PhysioNet/ Computing in Cardiology Challenge. *Physiological Measurement. 2021*, 2020, vol. 41 (12), 124003
- **ALDAY**. Classification of 12-lead ECGs: the PhysioNet/ Computing in Cardiology Challenge. *Physiological Measurement. 2021*, 2020, vol. 41 (12), 124003
- **ALDEY**. Classification of 12-lead ECGs: the PhysioNet/Computing in Cardiology Challenge. *Physiological Measurement. 2021*, 2020, vol. 41 (12), 124003